# EUROPÄISCHE PATENTANMELDUNG

(11) **EP 4 248 858 A1**
(43) Veröffentlichungstag der Anmeldung: **27.09.2023**
(21) Anmeldenummer: 23162479.2
(22) Anmeldetag: 16.03.2023
(51) Int. Cl.: A61B 5/11, A61B 5/1455, A61B 5/00, A61B 5/024

(54) **SCHLAF-/WACHERKENNUNG**

(30) Priorität: 22.03.2022 DE 102022106673
(71) Anmelder: Löwenstein Medical Technology S.A., 2557 Luxembourg (LU)
(72) Erfinder: Samie, Farzad, 76137 Karlsruhe (DE); Schwaibold, Matthias, 76228 Karlsruhe (DE)
(74) Vertreter: Marx, Thomas

(57) **Zusammenfassung**

Die Erfindung betrifft ein Verfahren zur Ermittlung von mindestens einer Schlafphase und/oder einer Wachphase für mindestens einen Zeitraum sowie ein System mit einer Steuereinheit zur Ermittlung von mindestens einer Schlafphase und/oder einer Wachphase für mindestens einen Zeitraum umfassend mindestens einen Sensor eingerichtet zur Erfassung mindestens eines Bewegungssignals , mindestens einen Sensor eingerichtet zur Erfassung mindestens eines Herzsignals, mindestens einen Sensor eingerichtet zur Erfassung mindestens eines Atmungssignals, mindestens eine Überwachungseinheit, die eingerichtet ist, ein Vorhandensein und/oder eine Qualität der erfassten Bewegungssignale und Herzsignale und Atmungssignale zu ermitteln und/oder zu bewerten sowie eine oder mehrere Klassifizierungseinheiten, die eingerichtet sind, auf Basis der Ermittlungen und Bewertungen der Überwachungseinheit aus den Bewegungssignalen und/oder den Herzsignalen und/oder den Atmungssignalen mindestens eine Schlafphase und/oder eine Wachphase zu ermitteln.

## Beschreibung

Eine Vielzahl an Menschen leiden unter einem schlechten Schlaf, der zu nachlassender Konzentration und verminderter geistiger Leistungsfähigkeit führt. Ein schlechter Schlaf kann ebenso für Störungen anderer Funktionen wie beispielsweise Muskelspannung, Atmung, Herzschlag, Blutdruck, Körpertemperatur, Hormone und Stoffwechsel verantwortlich sein. Die Ursachen für einen schlechten Schlaf können dabei vielfältig sein. Oftmals resultiert ein schlechter Schlaf aus einer Schlafstörung des Patienten. Ein schlechter Schlaf kann jedoch auch vorliegen, obwohl keine Schlafstörung ermittelt werden konnte. Um eine Aussage über die Schlafqualität eines Patienten treffen zu können und um mögliche Schlafstörungen oder Erkrankungen entsprechend beheben bzw. therapieren zu können, ist es notwendig, eine detaillierte Schlafdiagnostik durchzuführen.

Es ist Aufgabe der vorliegenden Erfindung, ein System bereitzustellen, das eine verbesserte, einfache und zuverlässige Aussage über den Schlaf eines Patienten liefert.

Diese Aufgabe wird gelöst mit einem System des Anspruchs 1 sowie mit einem Verfahren gemäß Anspruch 18 Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Die Erfindung betrifft ein System mit einer Steuereinheit zur Ermittlung von mindestens einer Schlafphase und/oder einer Wachphase für mindestens einen Zeitraum umfassend mindestens einen Sensor eingerichtet zur Erfassung mindestens eines Bewegungssignals, mindestens einen Sensor eingerichtet zur Erfassung mindestens eines Herzsignals, mindestens einen Sensor eingerichtet zur Erfassung mindestens eines Atmungssignals, mindestens eine Überwachungseinheit, die eingerichtet ist, ein Vorhandensein und/oder eine Qualität der erfassten Bewegungssignale und Herzsignale und Atmungssignale zu ermitteln und/oder zu bewerten, eine oder mehrere Klassifizierungseinheiten, die eingerichtet sind, auf Basis der Ermittlungen und Bewertungen der Überwachungseinheit aus den Bewegungssignalen und/oder den Herzsignalen und/oder den Atmungssignalen mindestens eine Schlafphase und/oder eine Wachphase zu ermitteln.

In manchen Ausführungsformen ist das System dadurch gekennzeichnet, dass die Überwachungseinheit eingerichtet ist, Bewegungssignale und/oder Herzsignale und/oder Atmungssignale von den Sensoren, zu empfangen.

In manchen Ausführungsformen ist das System dadurch gekennzeichnet, dass in der Überwachungseinheit mindestens ein Schwellenwert hinterlegt ist und dass die Qualität vorhandener Bewegungssignale und/oder Herzsignale und/oder Atmungssignale anhand des mindestens einen vordefinierten Schwellenwertes bewertet wird.

In manchen Ausführungsformen ist das System dadurch gekennzeichnet, dass für die Bewegungssignale und die Herzsignale und die Atmungssignale jeweils mindestens ein Schwellenwert hinterlegt ist, wobei die Qualität vorhandener Bewegungssignale und/oder Herzsignale und/oder Atmungssignale anhand ihrer jeweiligen Schwellenwerte bewertet werden.

In manchen Ausführungsformen ist das System dadurch gekennzeichnet, dass die Überwachungseinheit eingerichtet ist, vorhandene Bewegungssignale und/oder Herzsignale und/oder Atmungssignale, die über oder unter dem jeweiligen Schwellenwert liegen, zu selektieren.

In manchen Ausführungsformen ist das System dadurch gekennzeichnet, dass die Überwachungseinheit eingerichtet ist, die selektierten Bewegungssignale und/oder Herzsignale und/oder Atmungssignale weiterzuleiten.

In manchen Ausführungsformen ist das System dadurch gekennzeichnet, dass das System eine oder mehrere Extraktionseinheiten umfasst, die eingerichtet sind, selektierte Signale, von der Überwachungseinheit zu empfangen und aus den selektierten Bewegungssignalen mindestens ein Bewegungsmerkmal und/oder aus den selektierten Herzsignalen mindestens ein Herzmerkmal und/oder aus den selektierten Atmungssignalen mindestens ein Atmungsmerkmal zu ermitteln.

In manchen Ausführungsformen ist das System dadurch gekennzeichnet, dass die ermittelten Bewegungsmerkmale ausgewählt sind aus der Gruppe Bewegungszählung (Activity count), Nulldurchgangszählung (Zero-Crossing count), Median, Standardabweichung, Beschleunigungs-Minima, Beschleunigungs-Maxima.

In manchen Ausführungsformen ist das System dadurch gekennzeichnet, dass die ermittelten Herzmerkmale ausgewählt sind aus der Gruppe Herzfrequenz, Herzschlag, Herzschlagintervall, Herzfrequenzvariabilität, Pulsfrequenz, Pulswelle, Pulswellenamplitude, Spektrale Leistungsdichte, Sauerstoffsättigung (SpO₂ oder SaO₂), Plethysmogramm, Anstiegswinkel der Pulswellen, Abfallswinkel der Pulswellen, Verhältnisse aus Anstiegs- zu Abfallwinkel der Pulswellen, Dauern von Pulswellenanstiegen, Dauern von Pulswellenabfällen, Verhältnisse von Anstiegs- zu Abfallsdauern, Pulswellenmaxima, Pulswellenminima, PTT (Pulstransit-Zeit), CWF (continous wave fluctuation), Pulsamplitude.

In manchen Ausführungsformen ist das System dadurch gekennzeichnet, dass die ermittelten Atmungsmerkmale ausgewählt sind aus der Gruppe Atemfrequenz, Peakflow, Variabilität, Atemgasvolumen pro Atemzug, Atemgasfluss, Kontur des Atemgasflusses, inspiratorisches oder exspiratorisches Tidalvolumen, Atemzugsdauer, Inspirationsdauer, Exspirationsdauer, Leckage, Verhältnis von Inspirations- zu Exspirationsdauer, Atemminutenvolumen.

In manchen Ausführungsformen ist das System dadurch gekennzeichnet, dass die Extraktionseinheit eingerichtet ist, die extrahierten Bewegungsmerkmale und/oder Herzmerkmale und/oder Atmungsmerkmale weiterzuleiten.

In manchen Ausführungsformen ist das System dadurch gekennzeichnet, dass die Klassifizierungseinheiten eingerichtet sind, Signale von der Überwachungseinheit und/oder Merkmale von den Extraktionseinheiten zu empfangen.

In manchen Ausführungsformen ist das System dadurch gekennzeichnet, dass die Klassifizierungseinheiten eingerichtet sind, die Signale und/oder die Merkmale anhand mindestens einer statistischen Kennzahl statistisch auszuwerten.

In manchen Ausführungsformen ist das System dadurch gekennzeichnet, dass die statistische Kennzahl ausgewählt ist aus der Gruppe Minimum, Maximum, Mittelwert, Median, Standardabweichung, Varianz, Spannweite, Verteilung, Summe, Differenz.

In manchen Ausführungsformen ist das System dadurch gekennzeichnet, dass die Klassifizierungseinheiten eingerichtet sind, aus den Bewegungssignalen und/oder den Herzsignalen und/oder den Atmungssignalen und/oder aus deren statistischen Auswertungen mindestens eine Schlafphase und/oder eine Wachphase zu ermitteln.

In manchen Ausführungsformen ist das System dadurch gekennzeichnet, dass die Klassifizierungseinheiten eingerichtet sind, aus den Bewegungsmerkmalen und/oder den Herzmerkmalen und/oder den Atmungsmerkmalen und/oder deren statistischen Auswertungen mindestens eine Schlafphase und/oder eine Wachphase zu ermitteln.

In manchen Ausführungsformen ist das System dadurch gekennzeichnet, dass die Klassifizierungseinheit drei Klassifizierungsuntereinheiten umfasst, wobei die Steuereinheit eingerichtet ist, basierend auf den Ergebnissen der Überwachungseinheit in Abhängigkeit von dem Vorhandensein und/oder der Qualität der Signale, jeweils eine der drei Klassifizierungsuntereinheiten anzusteuern, wobei die erste Klassifizierungsuntereinheit angesteuert wird, wenn alle Signale in ausreichender Qualität vorhanden sind, wobei die zweite Klassifizierungsuntereinheit angesteuert wird, wenn nur die Bewegungssignale und die Herzsignale in ausreichender Qualität vorhanden sind, wobei die dritte Klassifizierungsuntereinheit angesteuert wird, wenn nur die Bewegungssignale in ausreichender Qualität vorhanden sind.

In manchen Ausführungsformen ist das System dadurch gekennzeichnet, dass die erste Klassifizierungsuntereinheit eingerichtet ist, Bewegungsmerkmale und Herzmerkmale und Atmungsmerkmale zu empfangen und Schlafphasen und/oder Wachphasen aus den Bewegungsmerkmalen und den Herzmerkmalen und den Atmungsmerkmalen zu ermitteln.

In manchen Ausführungsformen ist das System dadurch gekennzeichnet, dass die zweite Klassifizierungsuntereinheit eingerichtet ist, Bewegungsmerkmale und Herzmerkmale zu empfangen und Schlafphasen und/oder Wachphasen aus den Bewegungsmerkmalen und den Herzmerkmalen zu ermitteln.

In manchen Ausführungsformen ist das System dadurch gekennzeichnet, dass die dritte Klassifizierungsuntereinheit eingerichtet ist, Bewegungsmerkmale zu empfangen und Schlafphasen und/oder Wachphasen aus den Bewegungsmerkmalen zu ermitteln.

In manchen Ausführungsformen ist das System dadurch gekennzeichnet, dass der Zeitraum vorgebbar ist, wobei der Zeitraum wenigstens 10 Minuten, bevorzugt wenigstens eine Stunde, bevorzugt wenigstens 4 Stunden, besonders bevorzugt wenigstens 6 Stunden beträgt, wobei der Zeitraum beispielsweise der Dauer eines Nachtschlafs entspricht.

In manchen Ausführungsformen ist das System dadurch gekennzeichnet, dass der Zeitraum in ein oder mehrere Zeitperioden unterteilt ist, wobei die Zeitperioden wenigstens 10 Sekunden, bevorzugt wenigstens 20 Sekunden, besonders bevorzugt 30 Sekunden lang sind.

In manchen Ausführungsformen ist das System dadurch gekennzeichnet, dass das System eingerichtet und ausgebildet ist, anhand der Bewegungssignale und/oder der Herzsignale und/oder der Atmungssignale mindestens einer, bevorzugt jeder Zeitperiode eine Schlafphase und/oder eine Wachphase zuzuordnen.

In manchen Ausführungsformen ist das System dadurch gekennzeichnet, dass das System eingerichtet und ausgebildet ist, anhand der Bewegungsmerkmale und/oder der Herzmerkmale und/oder der Atmungsmerkmale mindestens einer, bevorzugt jeder analysierten Zeitperiode eine Schlafphase und/oder eine Wachphase zuzuordnen.

In manchen Ausführungsformen ist das System dadurch gekennzeichnet, dass in den Klassifizierungseinheiten mindestens ein Grenzwert hinterlegt ist und dass die Zuordnung der Schlafphasen und/oder Wachphasen zu den Zeitperioden anhand des mindestens einen vordefinierten Grenzwertes ermittelt wird.

In manchen Ausführungsformen ist das System dadurch gekennzeichnet, dass eine Schlafphase zu einer Zeitperiode ermittelt wird, wenn die Werte über dem einen oder den mehreren Grenzwerten liegen und dass eine Wachphase zu einer Zeitperiode ermittelt wird, wenn die Werte unter dem einen oder den mehreren Grenzwerten liegen, oder vice versa.

In manchen Ausführungsformen ist das System dadurch gekennzeichnet, dass die Zuordnung der Schlafphasen und/oder Wachphasen zu den Zeitperioden anhand von mehreren Grenzwerten ermittelt wird, wobei für jedes Merkmal jeweilige Grenzwerte in den Klassifizierungseinheiten hinterlegt und abrufbar sind.

In manchen Ausführungsformen ist das System dadurch gekennzeichnet, dass die Klassifizierungseinheiten eingerichtet sind, aus mindestens einem Merkmal bevorzugt aus mindestens zwei oder mehr unterschiedlichen Merkmalen die Schlafphasen und/oder Wachphasen je Zeitperiode zu ermitteln.

In manchen Ausführungsformen ist das System dadurch gekennzeichnet, dass die Analyse der unterschiedlichen Merkmale unterschiedlich gewichtet wird, wofür mindestens ein Gewichtwert in den Klassifizierungseinheiten hinterlegt und abrufbar ist, wobei bevorzugt für jedes Merkmal ein jeweiliger Gewichtwert hinterlegt ist.

In manchen Ausführungsformen ist das System dadurch gekennzeichnet, dass das System dynamisch ist, wobei die Steuereinheit während eines Zeitraum die drei Klassifizierungsuntereinheiten einmalig und/oder mehrmals ansteuert in Abhängigkeit von dem Vorhandensein und der Qualität der Signale.

In manchen Ausführungsformen ist das System dadurch gekennzeichnet, dass das System eingerichtet ist, basierend auf der Anzahl der Schlafphasen und/oder der Wachphasen eine Schlafqualität zu ermitteln.

In manchen Ausführungsformen ist das System dadurch gekennzeichnet, dass der Sensor ein Photoplethysmographie (PPG)-Sensor ist, der eingerichtet ist, PPG-Signale zu ermitteln, die Informationen bezüglich der Herztätigkeit liefern.

In manchen Ausführungsformen ist das System dadurch gekennzeichnet, dass der Sensor ein Aktigraphie-Sensor ist, der eingerichtet ist, Aktigraphie-Signale zu ermitteln, die Informationen bezüglich einer körperlichen Bewegung liefern.

In manchen Ausführungsformen ist das System dadurch gekennzeichnet, dass der Sensor ein Flow-Sensor ist, der eingerichtet ist, Flow-Signale zu ermitteln, die Informationen bezüglich der Atmungstätigkeit liefern.

In manchen Ausführungsformen ist das System dadurch gekennzeichnet, dass die Überwachungseinheit, die Extraktionseinheit und die Klassifizierungseinheit als eine oder mehrere Recheneinheiten ausgebildet sind, die eingerichtet sind, Messwerte aufzunehmen und/oder zu speichern und/oder auszuwerten und/oder zu bewerten und/oder zu selektieren und/oder weiterzuleiten und/oder zu verarbeiten, wobei in den Recheneinheiten, Datensätze wie beispielsweise Schwellenwerte und/oder Grenzwerte und/oder Regeln wie die Gewichtwerte hinterlegt sind, anhand derer die Messwerte und/oder die extrahierten Merkmale von den Recheneinheiten ausgewertet werden.

Gegenstand der Erfindung ist auch ein Verfahren zur Ermittlung von mindestens einer Schlafphase und/oder einer Wachphase für mindestens einen Zeitraum.

In einem ersten Schritt wird mindestens ein Signal mindestens eines Bewegungsparameters und/oder mindestens ein Signal mindestens eines Herzparameters und/oder mindestens ein Signal mindestens eines Atmungsparameters erfasst.

In einem weiteren Schritt wird das Vorhandensein der erfassten Bewegungssignale und Herzsignale und Atmungssignale ermittelt und/oder die Qualität der erfassten Bewegungssignale und Herzsignale und Atmungssignale bewertet.

In einem weiteren Schritt wird mindestens eine Schlafphase und/oder eine Wachphase aus den Bewegungssignalen und/oder den Herzsignalen und/oder den Atmungssignalen auf Basis der Ermittlungen und Bewertungen der Überwachungseinheit ermittelt.

In manchen Ausführungsformen ist das Verfahren dadurch gekennzeichnet, dass aus den Bewegungssignalen mindestens ein Bewegungsmerkmal und/oder aus den Herzsignalen mindestens ein Herzmerkmal und/oder aus den Atmungssignalen mindestens ein Atmungsmerkmal ermittelt wird.

In manchen Ausführungsformen ist das Verfahren dadurch gekennzeichnet, dass aus den Bewegungsmerkmalen und/oder den Herzmerkmalen und/oder den Atmungsmerkmalen mindestens eine Schlafphase und/oder eine Wachphase ermittelt wird.

In den Figuren sind Ausführungsbeispiele des erfindungsgemäßen Systems dargestellt. Es zeigen:
**Fig. 1** eine beispielhafte Vorrichtung, in die das erfindungsgemäße System zumindest teilweise integriert sein kann;
**Fig. 2** beispielhaft ermittelte Signalkurven, wobei die Signale eines Flows, einer Aktigraphie und eines (Photo-)Plethysmogramms graphisch über die Zeit t dargestellt sind.
**Fig. 3** einen beispielhaften schematischen Aufbau eines einfachen Ausführungsbeispiels des erfindungsgemäßen Systems, das drei Sensoren, eine Überwachungseinheit und eine Klassifizierungseinheit umfasst;
**Fig. 4** einen beispielhaften schematischen Aufbau eines weiteren Ausführungsbeispiels des erfindungsgemäßen Systems, wobei zusätzlich eine Extraktionseinheit umfasst ist, die zwischen die Überwachungseinheit und die Klassifizierungseinheit geschaltet ist;
**Fig. 5** eine beispielhaftes ermitteltes (Photo-)Plethysmogramm mit einer beispielhaften Darstellung extrahierter Herzmerkmale.
**Fig. 6** eine beispielhafte Darstellung eines extrahierten Herzmerkmals in einem Koordinatensystem, wobei auf der X-Achse die Herzschläge aufgetragen sind und auf der Y-Achse das Herzschlagintervall in Sekunden.

In den nachfolgenden Ausführungsbeispielen ist ein erfindungsgemäßes System 100 beschrieben. Weitere Merkmale und Vorteile der vorliegenden Erfindung werden in den nachfolgenden Beschreibungen von Ausführungsbeispielen anhand der Figuren deutlich. Die Erfindung ist nicht auf die dargestellten Ausführungsbeispiele beschränkt.

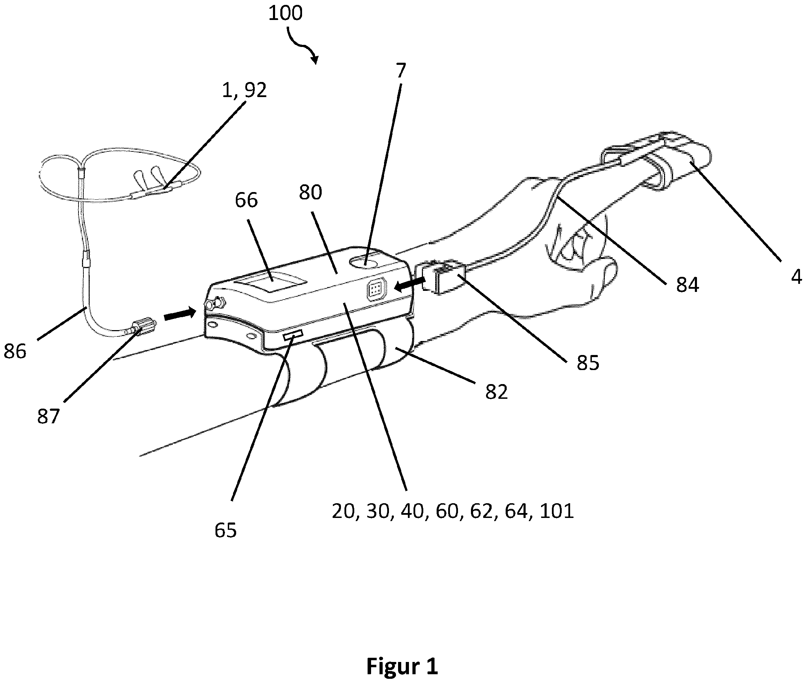

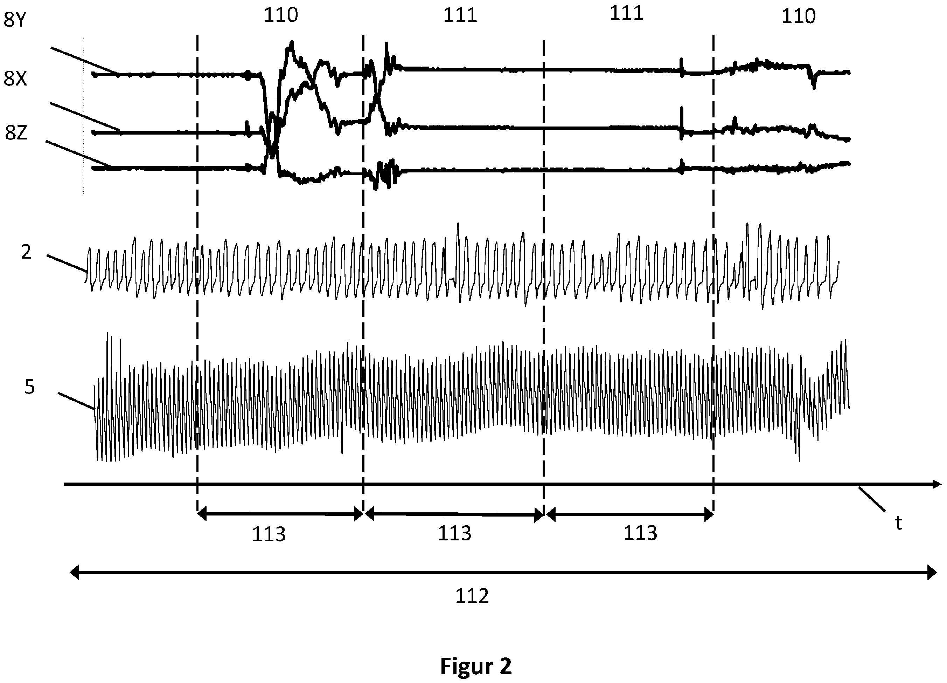

**Fig. 1** zeigt eine beispielhafte Vorrichtung 80, in die das erfindungsgemäße System 100 zumindest teilweise integriert sein kann. Das System 100 ist eingerichtet und ausgebildet, mindestens eine Schlafphase 110 und/oder eine Wachphase 111 für mindestens einen Zeitraum 112 zu ermitteln (siehe Fig. 2).

Das System 100 kann beispielsweise zumindest teilweise in eine oder mehrere Vorrichtungen 80 integriert sein, die beispielsweise am Körper eines Patienten oder Anwenders befestigt werden können. Patient und Anwender werden hierin als Synonyme verwendet und bezeichnen jedwedes Individuum, das das erfindungsgemäße System 100 zur Ermittlung von Schlafphasen 110 und/oder Wachphasen 111 nutzt bzw. an dem es angewendet wird. Die Vorrichtung 80 kann in manchen Ausführungsbeispielen auch nicht am Körper befestigt werden und beispielsweise als bettseitige Vorrichtung 80 ausgebildet sein. Die Vorrichtung 80 kann Daten kabelgebunden und/oder kabellos empfangen. Es ist somit auch denkbar, dass sich die Vorrichtung 80 unabhängig vom Standort des Patienten befindet und Daten kabellos empfängt (nicht gezeigt).

Bevorzugt wird die Vorrichtung 80 am Körper des Patienten befestigt. Die Befestigung der Vorrichtung 80 am Körper des Patienten kann beispielsweise über einen oder mehrere Vorrichtungsgurte 82 erfolgen. Der Vorrichtungsgurt 82 ist beispielsweise aus einem hautfreundlichen Kunststoff gefertigt. Eine andere Befestigung am Körper des Patienten ist auch möglich, beispielsweise über ein Aufkleben auf der Haut oder ähnliches. Die Vorrichtung 80 kann beispielsweise am Handgelenk eines Patienten befestigt werden. Eine solche Handgelenksvorrichtung 80 ist für den Patienten besonders angenehm zu tragen und stört den Schlaf nicht wesentlich.

Das System 100 umfasst mindestens drei Sensoren 1,4,7 zur nicht-invasiven Messung von mindestens einem Signal 2,5,8. Die mindestens drei Sensoren 1,4,7 sind eingerichtet und ausgebildet, Signale 2,5,8 aus mindestens drei unterschiedlichen Kategorien zu erfassen. Die Kategorien betreffen mindestens die körperliche Bewegung und die Herztätigkeit und die Atmungstätigkeit des Patienten.

Das System 100 umfasst mindestens einen Sensor 7 eingerichtet zur Erfassung mindestens eines Bewegungssignals 8. Der Sensor 7 ist eingerichtet und ausgebildet, Bewegungssignale 8 nicht-invasiv zu ermitteln. Der Sensor 7 kann beispielsweise ein Aktigraphie-Sensor 7 sein. Das Bewegungssignal 8 kann beispielsweise ein Aktigraphie-Signal 8 sein. In diesem konkreten Ausführungsbeispiel kann der Aktigraphie-Sensor 7 eingerichtet sein, Aktigraphie-Signale 8 zu ermitteln, die Informationen bezüglich einer körperlichen Bewegung liefern.

Das Bewegungssignal 8 kann in der beispielhaften Ausführungsform gemäß Fig. 1 über einen Sensor 7 am Handgelenk ermittelt werden. Es ist auch denkbar, dass der Sensor 7 an einer anderen Stelle des Körpers angebracht ist. Mehrere Sensoren 7 an unterschiedlichen Stellen des Körpers des Patienten sind auch denkbar, um differenziertere Aussagen über die körperliche Bewegung zu ermitteln.

Der Sensor 7 kann beispielsweise in eine Vorrichtung 80 integriert sein, die beispielsweise am Handgelenk getragen wird. In einer alternativen Ausführungsform ist es denkbar, dass das Bewegungssignal 8 alternativ oder zusätzlich am Oberkörper des Patienten gemessen wird. Hierfür kann der Sensor 7 in einer Vorrichtung 80 angeordnet sein, die über einen Vorrichtungsgurt 82 am Abdomen und/oder am Thorax befestigt werden. Alternativ oder zusätzlich kann der Sensor 7 auch beispielsweise an den unteren Extremitäten und/ oder am Kopf befestigt werden (nicht gezeigt).

Das System 100 kann in manchen Ausführungsformen mehrere Sensoren 7 umfassen, die die körperliche Bewegung an unterschiedlichen Stellen des Körpers des Patienten erfassen können. Die erfassten körperlichen Bewegungen können ausgewählt sein aus der Gruppe Arm-Bewegungen, Handgelenk-Bewegungen, Hand-Bewegungen, Bein-Bewegungen, Fuß-Bewegungen, Fußgelenk-Bewegungen, Thorax-Bewegungen, Abdomen-Bewegungen, Kopf-Bewegungen, Augen-Bewegungen.

Der Aktigraphie-Sensor 7 kann ein oder mehrere Aktigraphie-Signale 8 erfassen. Die Aktigraphie-Signale 8 können in der Vorrichtung 80 ermittelt werden. Die Aktigraphie-Signale 8 können auch außerhalb der Vorrichtung 80 ermittelt und beispielsweise in die Vorrichtung 80 übermittelt werden. Die Übermittlung der Aktigraphie-Signale 8 erfolgt zu Recheneinheiten 20, 30, 40 zur Weiterverarbeitung der Aktigraphie-Signale 8. Diese Recheneinheiten 20, 30, 40 können beispielsweise in der Vorrichtung 80 angeordnet sein.

Die Aktigraphie-Signale 8 können beispielsweise als Aktigraphiekurven über die Zeit t aufgezeichnet werden. In einem beispielhaften Ausführungsbeispiel können beispielsweise drei Aktigraphie-Kurven 8X, 8Y, 8Z über die Zeit t aufgezeichnet werden (siehe Fig. 2). Die drei Aktigraphiekurven beschreiben in diesem Ausführungsbeispiel die Beschleunigung, der der Aktigraphie-Sensor 7 in den drei Bewegungsrichtungen X,Y,Z ausgesetzt ist. Hierbei könnte die X-Richtung zum Beispiel eine Bewegung nach links oder rechts darstellen, die Y-Richtung eine Bewegung nach vorn oder hinten und die Z-Richtung eine Bewegung nach oben oder unten.

Das System umfasst zudem mindestens einen Sensor 4 eingerichtet zur Erfassung mindestens eines Herzsignals 5. Der Sensor 4 ist eingerichtet und ausgebildet, Herzsignale 5 nicht-invasiv zu ermitteln. Bevorzugt wird ein Plethysmogramm, beispielsweise ein Photoplethysmogramm mit einem Pulsoximeter und/oder einem Pulsspektrometer ermittelt und aufgezeichnet. Der Sensor 4 kann beispielsweise ein Photoplethysmographie-Sensor, kurz ein PPG-Sensor 4, sein. Das Herzsignal 5 kann beispielsweise ein Photoplethysmographie-Signal, kurz ein PPG-Signal 5, sein. In diesem konkreten Ausführungsbeispiel kann der PPG-Sensor 4 eingerichtet sein, PPG-Signale 5 zu ermitteln, die Informationen bezüglich der Herztätigkeit liefern.

Das Herzsignal 5 kann in dieser beispielhaften Ausführungsform gemäß Fig. 1 am Finger gemessen werden. Es ist auch möglich, dass das Herzsignal 5 am Kopf, beispielsweise am Ohrläppchen, gemessen wird. Denkbar ist auch eine Messung am Fuß, am Zeh, am Oberkörper, am Handgelenk oder an einer anderen geeigneten Stelle des Körpers (nicht gezeigt).

Der PPG-Sensor 4 kann in die Vorrichtung 80 integriert sein. In dem konkreten Ausführungsbeispiel gemäß Fig. 1 ist der PPG-Sensor 4 nicht in die Vorrichtung 80 integriert. Beispielsweise befindet sich der PPG-Sensor 4 in einem Pulsoximeter und/oder einem Pulsspektrometer, der an einem Finger befestigt wird. Entsprechende Pulsoximeter und Pulsspekrometer sind bekannt. Der Sensor 4 kann beispielsweise pulsoximetrische Signale 5 erfassen, wie beispielsweise eine Sauerstoffsättigung (SpOz) des Blutes und/oder eine Pulsfrequenz des Patienten. Der Pulsoxymetriesensor 4 kann im Wesentlichen zumindest eine, bevorzugt mindestens zwei Leuchtdioden und eine Empfängerdiode umfassen. Über die Messung der Lichtabsorption bzw. der Lichtremission bei Durchleuchtung der Haut können die pulsoximetrischen Signale 5 erfasst werden.

Beispielsweise kann das PPG-Signal 5 am Finger ermittelt werden. Das PPG-Signal 5 kann sodann über eine Kabelverbindung 84 übermittelt werden. Die Kabelverbindung 84 kann über einen Anschluss 85 an die Vorrichtung 80 angeschlossen werden. Eine kabellose Übermittlung der PPG-Signale 5, beispielsweise über Bluetooth ist auch denkbar. Beispielsweise wird das PPG-Signal 5 in die Vorrichtung 80 übermittelt, in der Recheneinheiten 20, 30, 40 zur Weiterverarbeitung des PPG-Signals 5 angeordnet sein können.

Das System 100 umfasst zudem mindestens einen Sensor 1 eingerichtet zur Erfassung mindestens eines Atmungssignals 2. Der Sensor 1 ist eingerichtet und ausgebildet, Atmungssignale 2 nicht-invasiv zu ermitteln. Die Atmungssignale 5 sind ausgewählt aus der Gruppe: Flow (Atemgasfluss), Volumen (Atemgasvolumen), Druck.

Der Sensor 1 kann beispielsweise ein Flow-Sensor 1 sein. Das Atmungssignal 2 kann beispielsweise ein Flow-Signal 2 sein. In diesem konkreten Ausführungsbeispiel kann der Flow-Sensor 1 eingerichtet sein, Flow-Signale 2 zu ermitteln, die Informationen bezüglich der Atmungstätigkeit liefern.

Das Signal 2 kann über ein Patienteninterface 92 ermittelt werden. Das Signal 2 wird in dem konkreten Ausführungsbeispiel gemäß Fig. 1 über ein Patienteninterface 92 ermittelt, das als Staudruck-Nasenbrille 92 ausgebildet ist (Darstellung nicht maßstabsgetreu). Die Staudruck-Nasenbrille 92 kann in Verbindung mit einem in die Vorrichtung 80 integrierten Sensor 1 beispielsweise den Flow und/oder den Druck und/oder das Volumen erfassen. Das Signal 2 kann über eine Schlauchverbindung 86 übermittelt werden. Die Schlauchverbindung 86 kann über einen Anschluss 87 an die Vorrichtung 80 angeschlossen werden. Der Anschluss 87 kann beispielsweise als Luer-Lock-Anschluss ausgebildet sein. Beispielsweise wird das Flow-Signal 2 in die Vorrichtung 80 übermittelt, in der Recheneinheiten 20, 30, 40 zur Weiterverarbeitung des Flow-Signals 2 angeordnet sein können.

Die Bewegungssignale 8 und/oder Herzsignale 5 und/oder Atmungssignale 2 können aufgezeichnet werden und beispielsweise anhand von Signalkurven über die Zeit t dargestellt werden. Zu diesem Zweck kann das System 100 eine Anzeigeeinrichtung 66 umfassen. Die Anzeigeeinrichtung 66 kann einen Monitor umfassen. Der Monitor kann beispielsweise in die Vorrichtung 80 integriert sein. In einer bevorzugten Ausführungsform kann der Monitor unabhängig von der Vorrichtung 80 eingerichtet und ausgebildet sein und die Daten vom System 100 empfangen und darstellen.

Neben den mindestens drei Sensoren 1,4,7 umfasst das System 100 mindestens eine Überwachungseinheit 20 und mindestens eine Klassifizierungseinheit 40. Die Überwachungseinheit 20 ist eingerichtet, ein Vorhandensein und/oder eine Qualität der erfassten Bewegungssignale 8 und Herzsignale 5 und Atmungssignale 2 zu ermitteln und/oder zu bewerten. Die eine oder mehreren Klassifizierungseinheiten 40 sind eingerichtet, auf Basis der Ermittlungen und Bewertungen der Überwachungseinheit 20 aus den Bewegungssignalen 8 und/oder den Herzsignalen 5 und/oder den Atmungssignalen 2 mindestens eine Schlafphase 110 und/oder eine Wachphase 111 zu ermitteln. Neben der Überwachungseinheit 20 und der Klassifizierungseinheit 40 kann das System 100 zudem mindestens eine Extraktionseinheit 30 umfassen. Die Überwachungseinheit 20, die Extraktionseinheit 30 und die Klassifizierungseinheit 40 sind als eine oder mehrere Recheneinheiten ausgebildet, die eingerichtet sind, Messwerte 2,5,8 aufzunehmen und/oder zu speichern und/oder auszuwerten und/oder zu bewerten und/oder zu selektieren und/oder weiterzuleiten und/oder zu verarbeiten.

In dem konkreten Ausführungsbeispiel nach Fig. 1 können die Recheneinheiten, nämlich die Überwachungseinheit 20, die Extraktionseinheit 30 und die Klassifizierungseinheit 40 in der Vorrichtung 80 hinterlegt sein. Eine externe Hinterlegung, beispielsweise in externen Computersystemen, Servern, Clouds ist ebenfalls denkbar.

Das System 100 umfasst zudem eine Steuereinheit 101. Die Steuereinheit 101 ist eingerichtet und ausgebildet, die unterschiedlichen Recheneinheiten 20, 30, 40 des Systems 100 zu steuern. Die Steuereinheit 101 kann ebenfalls in der Vorrichtung 80 oder extern hinterlegt sein.

Das System 100 kann mindestens eine Speichereinheit 60 umfassen. Die Speichereinheit 60 kann ebenfalls in der Vorrichtung 80 oder extern hinterlegt sein. Die Speichereinheit 60 kann eingerichtet sein, mindestens ein Signal zwischenzuspeichern und für eine Weiterverarbeitung zur Verfügung zu stellen. Die Speichereinheit 60 kann eingerichtet sein, erfasste und/oder ermittelte Daten für eine spätere Auslesung zu speichern. In der Speichereinheit 60 können darüber hinaus vordefinierte Datensätze wie beispielsweise Schwellenwerte 22 und/oder Grenzwerte 42 und/oder Regeln hinterlegt sein, anhand derer Messwerte bzw. Signale 2,5,8 und/oder aus den Signalen 2,5,8 extrahierte Merkmale 3,6,9 von den Recheneinheiten 20,30,40 ausgewertet werden.

Das System 100 kann eine Energieversorgung 62 umfassen. Die Energieversorgung 62 kann über Batterien und/oder Akkumulatoren erfolgen. Alternativ oder zusätzlich kann die Energieversorgung 62 extern über Netzkabel erfolgen.

Das System 100 kann eine Datenübertragungseinrichtung 64 umfassen. Über die Datenübertragungseinrichtung 64 kann die Eingabe und/oder Ausgabe von Daten erfolgen. Die Datenübertragung kann kabelgebunden und/oder drahtlos erfolgen. Die Datenübertragung kann über eine Schnittstelle 65 erfolgen. Die Schnittstelle 65 kann kabelgebunden, als Infrarot-Schnittstelle, als Bluetooth-Schnittstelle oder als USB-Schnittstelle eingerichtet sein. Über die Schnittstelle 65 können beispielsweise Mobilfunk- oder Nahfeldfunkdaten und/oder WLAN und/oder Bluetooth und/oder Netzwerkdaten empfangen oder gesendet werden.

**Fig. 2** zeigt beispielhaft ermittelte Signalkurven, wobei die Signale eines Flows 2, einer Aktigraphie 8 und eines (Photo-)Plethysmogramms 5 graphisch über die Zeit t dargestellt sind.

Die ermittelten Signale 2,5,8 können mittels Signalkurven über die Zeit t graphisch dargestellt werden. Die Signalkurven können beispielsweise über die Anzeigeeinrichtung 66 ausgegeben und angezeigt werden.

Das System 100 ist eingerichtet und ausgebildet, dass ein Zeitraum 112 vorgebbar ist. Der Patient oder medizinisches Personal kann den Zeitraum 112 vorgeben. Der Start und das Ende des Zeitraumes 112 kann voreingestellt werden. Der Zeitraum 112 beträgt wenigstens 10 Minuten. Beispielsweise beträgt der Zeitraum 112 wenigstens eine Stunde, bevorzugt wenigstens 2 Stunden, besonders bevorzugt wenigstens 6 Stunden. In einer vorteilhaften Ausgestaltung entspricht der Zeitraum 112 beispielsweise der Dauer eines Nachtschlafs. Fig. 2 zeigt einen beispielhaften Zeitraum 112, der nicht einem Nachtschlaf entspricht. In Fig. 2 ist ein Ausschnitt eines Zeitraums 112 dargestellt.

Der Zeitraum 112 kann in ein oder mehrere Zeitperioden 113 unterteilt werden. Die Zeitperioden 113 sind wenigstens 10 Sekunden, bevorzugt wenigstens 20 Sekunden lang. In einem konkreten Ausführungsbeispiel ist die Zeitperiode 113 30 Sekunden lang. Das bedeutet, dass die Zeitperioden 113 beispielsweise jeweils 30 Sekunden umfassen.

Das System 100 ist eingerichtet und ausgebildet, anhand der Bewegungssignale 8 und/oder der Herzsignale 5 und/oder der Atmungssignale 2 mindestens einer Zeitperiode 113 eine Schlafphase 110 und/oder eine Wachphase 111 zuzuordnen. Bevorzugt ordnet das System 100 jeder Zeitperiode 113 des Zeitraumes 112 eine Schlafphase 110 und/oder eine Wachphase 111 zu. Durch die Auswahl der drei Signalkategorien 8,5,2 stehen erfindungsgemäß stets ausreichende Signale 2,5,8 zur Verfügung, um bevorzugt jeder Zeitperiode 113 eine Schlafphase 110 und/oder eine Wachphase 111 zuzuordnen. In Fig. 2 ist beispielhaft dargestellt, dass auf eine Zeitperiode 113, der das System 100 eine Schlafphase 110 zuordnet, zwei Zeitperioden 113 folgen, der das System 100 jeweils eine Wachphase 111 zuordnet. Auf die beiden Wachphasen 111 folgt wiederrum eine Zeitperiode 113, der das System 100 eine Schlafphase 110 zuordnet.

Auf diese Weise kann das System 100 bevorzugt allen Zeitperioden 113 des gesamten Zeitraumes 112 Schlafphasen 110 und/oder Wachphasen 111 zuordnen. Durch das erfindungsgemäße System 100 mit den mindestens drei Sensoren 1,4,7 wird ein besonders zuverlässiges System 100 zur Ermittlung von Schlafphasen 110 und/oder Wachphasen 111 bereitgestellt.

Ziel der Erfindung ist es primär nicht, eine konkrete schlafbezogene Erkrankung oder Störung zu diagnostizieren. Ziel der Erfindung ist es vielmehr, eine detaillierte und zuverlässige Ermittlung der Schlafphasen 110 und Wachphasen 111 innerhalb eines Zeitraums 112 zu gewährleisten. Der Zeitraum 112 umfasst hierbei bevorzugt mindestens einen Nachtschlaf eines Patienten. Durch die Auswahl aus mindestens drei Signalen, nämlich mindestens einem Bewegungssignal 8, mindestens einem Herzsignal 5 und mindestens einem Atmungssignal 2 ist eine besonders sichere Ermittlung der Schlafphasen 110 und Wachphasen 111 gegeben.

Im Umkehrschluss ist es allerdings möglich, durch die detaillierte und zuverlässige Ermittlung der Schlafphasen 110 und Wachphasen 111 innerhalb des Zeitraums 112 auch Rückschlüsse auf konkrete schlafbezogene Erkrankungen oder Störungen oder auf eine Schlafqualität 120 zu ziehen.

Das System 100 ist somit insbesondere auch eingerichtet, basierend auf der Anzahl der Schlafphasen 110 und/oder der Wachphasen 111 eine Schlafqualität 120 zu ermitteln. Die Schlafqualität 120 kann eine Aussage darüber geben, ob der Schlaf einer oder mehrerer vergangener Zeiträume 112 insgesamt ein "guter Schlaf" oder ein "schlechter Schlaf" war.

Die Schlafqualität 120 kann beispielsweise als Schlafqualitätsindex ausgegeben werden. Der Schlafqualitätsindex kann beispielsweise in Form einer Zahl, eines Symbols, einer Graphik oder dergleichen ausgegeben werden und somit benutzerfreundlich und einfach auszulesen sein. Somit können auch Anwender ohne medizinische Fachkenntnis eine Aussage über die Schlafqualität 120 durch das System 100 erhalten, wobei ein niedriger Schlafqualitätsindex auf einen schlechteren Schlaf und ein höherer Schlafqualitätsindex auf einen besseren Schlaf hindeutet. Dem System 100 ist somit leicht zu entnehmen, wie die Schlafqualität 120 eines oder mehrerer zurückliegender Zeiträume 112 war.

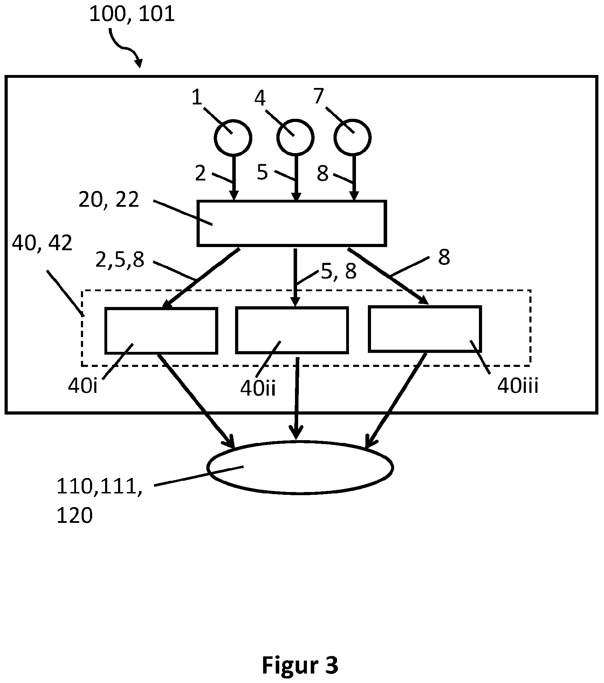

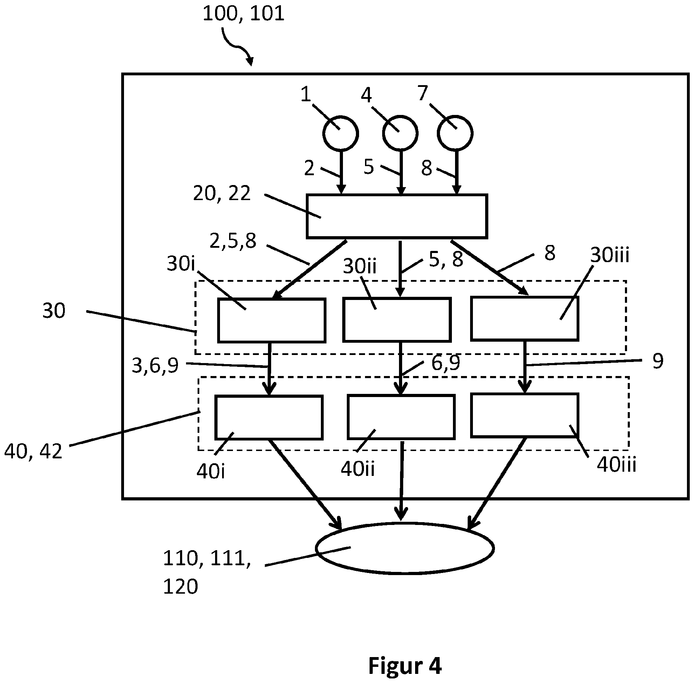

**Fig. 3** zeigt einen beispielhaften schematischen Aufbau eines einfachen Ausführungsbeispiels des erfindungsgemäßen Systems 100, das drei Sensoren 1,4,7, eine Überwachungseinheit 20 und eine Klassifizierungseinheit 40 umfasst. **Fig. 4** zeigt einen beispielhaften schematischen Aufbau eines weiteren Ausführungsbeispiels des erfindungsgemäßen Systems 100, wobei zusätzlich eine Extraktionseinheit 30 umfasst ist, die zwischen die Überwachungseinheit 20 und die Klassifizierungseinheit 40 geschaltet ist.

In den konkreten Ausführungsbeispielen gemäß Fig. 3 und Fig. 4 erfassen die drei Sensoren, nämlich beispielsweise der Flow-Sensor 1, der PPG-Sensor 4 und der Aktigraphie-Sensor 7 jeweils Flow-Signale 2, PPG-Signale 5 und Aktigraphie-Signale 8. Die erfassten Signale 2,5,8 werden an die Überwachungseinheit 20 weitergeleitet. Die Weiterleitung der erfassten Signale 2,5,8 kann entweder kabelgebunden und/oder kabellos erfolgen.

Die Überwachungseinheit 20 ist eingerichtet, Bewegungssignale 8 und/oder Herzsignale 5 und/oder Atmungssignale 2 von den Sensoren 1,4,7 zu empfangen. Die Überwachungseinheit 20 ermittelt sodann, welche Signale 2,5,8 vorhanden sind. Zudem bewertet die Überwachungseinheit 20 die Qualität der erfassten Signale 2,5,8.

Es hat sich gezeigt, dass in einem zu messenden Zeitraum 112 nicht immer alle Signale 2,5,8 zu erfassen oder zu messen sind. Zudem hat sich gezeigt, dass nicht immer alle Signale 2,5,8 in einer ausreichenden Qualität vorhanden sind. Das kann beispielsweise daran liegen, dass sich der Patient während des zu messenden Zeitraumes 112 bewegt, wodurch die Sensoren 1,4,7 eine ungünstige Positionsänderung erfahren können oder Übertragungswege der Signale 1,4,7 unterbrochen werden können.

Es hat sich gezeigt, dass das Bewegungssignal 8 in der Regel und bei sachgemäßen Gebrauch immer in einer ausreichenden Qualität vorhanden ist.

Das PPG-Signal 5 ist zumeist in einer ausreichenden Qualität vorhanden. Allerdings kann das PPG-Signal 5 während des zu messenden Zeitraumes 112 auch fehlen, zum Beispiel, wenn der PPG-Sensor 4 verrutscht oder gar ganz vom Finger abrutscht. Ein ungünstiges Verrutschen des PPG-Sensors 4 kann prinzipiell durch erneute Patientenbewegung wieder rückgängig gemacht werden und das Fehlen des PPG-Signals 5 könnte nur einen bestimmten Teil des Zeitraumes 112 betreffen und somit reversibel sein. Wenn der PPG-Sensor 4 jedoch unbemerkt abrutscht und somit dauerhaft entfernt wäre, wäre das Fehlen des PPG-Signals 5 dauerhaft und nicht reversibel.

Das Flow-Signal 2 ist während eines Zeitraums 112 nicht immer ermittelbar bzw. nicht immer in ausreichender Qualität zu erfassen. Beim Atmungssignal 2 können sich Patientenbewegungen am stärksten negativ auf die Messgenauigkeit auswirken. Zum Beispiel kann das Patienteninterface 92 im Schlaf unbemerkt verrutschen und/oder die Schlauchverbindung 86 kann unterbrochen werden. Beispielsweise kann die Schlauchverbindung 86 durch knicken oder quetschen nicht mehr in der Lage sein, den Flow zu transportieren. Diese Unterbrechungen können in der Regel reversibel sein und während des Zeitraumes 112 häufiger auftreten.

Um das Vorhandensein der Signale 2,5,8 zu ermitteln und/oder die Qualität der erfassten Signale 2,5,8 zu bewerten, kann in der Überwachungseinheit 20 mindestens ein Schwellenwert 22 hinterlegt sein. Sodann kann die Qualität der Bewegungssignale 8 und/oder Herzsignale 5 und/oder Atmungssignale 2 anhand des mindestens einen vordefinierten Schwellenwertes 22 bewertet werden. Bevorzugt ist zu jedem Signal 2,5,8 jeweils mindestens ein vordefinierter Schwellenwert 22 hinterlegt.

Bevorzugt ist für jede Signalkategorie, also für die Bewegungssignale 8 und die Herzsignale 5 und die Atmungssignale 2 jeweils mindestens ein Schwellenwert 22 hinterlegt. Die Qualität vorhandener Bewegungssignale 8 und/oder Herzsignale 5 und/oder Atmungssignale 2 kann somit jeweils anhand des entsprechenden Schwellenwerts 22 bewertet werden. Die Bewertung der Signale 2,5,8 erfolgt entweder anhand einer Überschreitung oder anhand einer Unterschreitung des entsprechenden Schwellenwertes 22. Auf Grundlage der Bewertung der Signale 2,5,8 anhand der Überschreitung oder Unterschreitung des entsprechenden Schwellenwertes 22 kann eine Selektierung der Signale erfolgen. Die Selektion kann über die Überwachungseinheit 20 erfolgen.

Die Überwachungseinheit 20 kann eingerichtet sein, vorhandene Bewegungssignale 8 und/oder Herzsignale 5 und/oder Atmungssignale 2, die über oder unter dem jeweiligen Schwellenwert 22 liegen, zu selektieren. Die Überwachungseinheit 20 kann ferner eingerichtet sein, die selektierten Bewegungssignale 8 und/oder Herzsignale 5 und/oder Atmungssignale 2 weiterzuleiten.

Die Weiterleitung der selektierten Bewegungssignale 8 und/oder Herzsignale 5 und/oder Atmungssignale 2 kann in einem einfachen Ausführungsbeispiel direkt an die Klassifizierungseinheit 40 erfolgen (siehe Fig. 3).

Die Weiterleitung der selektierten Bewegungssignale 8 und/oder Herzsignale 5 und/oder Atmungssignale 2 kann in einem bevorzugten Ausführungsbeispiel auch an die Extraktionseinheit 30 erfolgen (siehe Fig. 4).

Aus Fig.3 wird ersichtlich, dass das System 100 eine oder mehrere Klassifizierungseinheiten 40 umfasst, die in einem einfachen Ausführungsbeispiel eingerichtet sein kann, aus den Bewegungssignalen 8 und/oder den Herzsignalen 5 und/oder den Atmungssignalen 2 mindestens eine Schlafphase 110 und/oder eine Wachphase 111 zu ermitteln. In einer vorteilhaften Ausgestaltung kann die Klassifizierungseinheit 40 eingerichtet sein, aus statistischen Auswertungen der Bewegungssignale 8 und/oder der Herzsignale 5 und/oder der Atmungssignale 2 mindestens eine Schlafphase 110 und/oder eine Wachphase 111 zu ermitteln.

Das System 100 kann eine erste Klassifizierungsuntereinheit 40i umfassen, die eingerichtet sein kann, Bewegungssignale 8 und Herzsignale 5 und Atmungssignale 2 von der Überwachungseinheit 20 zu empfangen und Schlafphasen 110 und/oder Wachphasen 111 direkt aus den Bewegungssignalen 8 und den Herzsignalen 5 und den Atmungssignalen 2 zu ermitteln. Das System 100 kann darüber hinaus eine zweite Klassifizierungsuntereinheit 40ii umfassen, die eingerichtet sein kann, Bewegungssignale 8 und Herzsignale 5 von der Überwachungseinheit 20 zu empfangen und Schlafphasen 110 und/oder Wachphasen 111 aus den Bewegungssignalen 8 und den Herzsignalen 5 zu ermitteln. Das System 100 kann ferner eine dritte Klassifizierungsuntereinheit 40iii umfassen, die eingerichtet sein kann, lediglich Bewegungssignale 8 von der Überwachungseinheit 20 zu empfangen und Schlafphasen 110 und/oder Wachphasen 111 aus den Bewegungssignalen 8 zu ermitteln.

Die Klassifizierungseinheit 40 kann somit mindestens drei Untereinheiten 40i, 40ii, 40iii umfassen, so dass stets gewährleistet ist, dass aus den zur Verfügung stehenden Signalen 2, 5, 8 jeweils eine Schlafphase 110 oder eine Wachphase 111 je Zeitperiode 113 ermittelt werden kann. In manchen Ausführungsformen können auch mehr als drei Untereinheiten der Klassifizierungseinheit 40 vorgesehen sein, die weitere mögliche vorhandene Signalkombinationen verarbeiten können.

Die Ansteuerung der Untereinheiten 40i, 40ii, 40iii kann durch die Überwachungseinheit 20 erfolgen. Die Steuerung kann auch durch die Klassifizierungseinheit 40 selbst erfolgen. In einer beispielhaften Ausführungsform ist vorgesehen, dass die übergeordnete Steuereinheit 101 registriert, um welche Kombination an Signalen 2, 5, 8 es sich handelt und daraufhin die richtige Untereinheit 40i, 40ii oder 40iii ansteuert. Ansteuern bedeutet in diesem Sinne, dass die jeweilige Untereinheit 40i, 40 ii oder 40iii die entsprechenden Signale 2 und/oder 5 und/oder 8 zur Weiterverarbeitung erhält.

Das System 100 kann somit dynamisch ausgebildet sein, wobei die Steuereinheit 101 während eines Zeitraum 112 die Klassifizierungsuntereinheiten 40i, 40ii, 40iii einmalig und/oder mehrmals ansteuert in Abhängigkeit von dem Vorhandensein und der Qualität der Signale 2,5,8. Bevorzugt erfolgt die Ansteuerung und somit die Auswahl der entsprechenden Untereinheit 40i, 40ii, 40iii für jede einzelne Zeitperiode 113 von neuem.

**Fig. 4** zeigt ein bevorzugtes Ausführungsbeispiel mit mindestens einer Extraktionseinheit 30. In diesem Ausführungsbeispiel können die selektierten Bewegungssignale 8 und/oder Herzsignale 5 und/oder Atmungssignale 2 an die Extraktionseinheit 30 weitergeleitet werden (siehe Fig. 4). Die Extraktionseinheit 30 ist eingerichtet, die selektierten Signale 2,5,8 zu empfangen. Die Extraktionseinheit 30 ist eingerichtet, aus den selektierten Bewegungssignalen 8 mindestens ein Bewegungsmerkmal 9 und/oder aus den selektierten Herzsignalen 5 mindestens ein Herzmerkmal 6 und/oder aus den selektierten Atmungssignalen 2 mindestens ein Atmungsmerkmal 3 zu ermitteln. Die Extraktionseinheit 30 ist weiterhin eingerichtet, die extrahierten Bewegungsmerkmale 9 und/oder Herzmerkmale 6 und/oder die Atmungsmerkmale 3 weiterzuleiten.

Die Extraktionseinheit 30 kann in manchen Ausführungsformen in mindestens drei Untereinheiten 30i, 30ii und 30iii untergliedert sein. Die Extraktionsuntereinheiten 30i, 30ii, 30iii können jeweils eingerichtet sein, eine bestimmte Kombination an Signalen 2,5,8 von der Überwachungseinheit 20 zu empfangen. Die erste Extraktionsuntereinheit 30i kann eingerichtet sein, selektierte Bewegungssignale 8 und Herzsignale 5 und Atmungssignale 2 zu empfangen. Die erste Extraktionseinheit 30i wird sodann angesteuert, wenn alle drei Signale 2,5,8 in ausreichender Qualität vorhanden sind. Die zweite Extraktionsuntereinheit 30ii kann eingerichtet sein, selektierte Bewegungssignale 8 und Herzsignale 5 zu empfangen. Die zweite Extraktionseinheit 30ii wird sodann angesteuert, wenn nur zwei Signale, nämlich die Bewegungssignale 8 und die Herzsignale 5 in ausreichender Qualität vorhanden sind. Die dritte Extraktionsuntereinheit 30iii kann eingerichtet sein, selektierte Bewegungssignale 8 zu empfangen. Die dritte Extraktionseinheit 30iii wird sodann angesteuert, wenn nur das Bewegungssignal 8 in ausreichender Qualität vorhanden ist. In manchen Ausführungsformen können auch mehr als drei Untereinheiten der Extraktionseinheit 30 vorgesehen sein, die weitere mögliche vorhandene Signalkombinationen verarbeiten können.

Aus der Menge der Signaldaten 2,5,8 kann die Extraktionseinheit 30 und/oder ihre Untereinheiten 30i, 30ii, 30iii, die als Recheneinheiten ausgebildet sind, mit Hilfe einer Mustererkennung Regelmäßigkeiten, Wiederholungen, und/oder Gesetzmäßigkeiten erkennen und entsprechende mathematische Merkmale 3,6,9 extrahieren.

Die Extraktionseinheit 30 und/oder ihre Untereinheiten 30i, 30ii, 30iii sind somit eingerichtet, aus den Signalen 2,5,8 mindestens ein Merkmal 3,5,8 zu ermitteln und/oder zu extrahieren.

Die Extraktionseinheit 30 kann eingerichtet sein, mindestens aus den selektierten Bewegungssignalen 8 mindestens ein Bewegungsmerkmal 9 zu ermitteln. Die Extraktionseinheit 30 kann eingerichtet sein, mindestens aus den selektierten Herzsignalen 5 mindestens ein Herzmerkmal 6 zu ermitteln. Die Extraktionseinheit 30 kann eingerichtet sein, mindestens aus den selektierten Atmungssignalen 2 mindestens ein Atmungsmerkmal 3 zu ermitteln.

In der Extraktionseinheit 30 kann wenigstens ein Algorithmus hinterlegt sein, mittels dem aus einem oder mehreren Signalen 2,5,8 mindestens ein Merkmal 3,6,9 berechnet werden kann.

In dem konkreten Ausführungsbeispiel nach Fig. 4 ist die erste Extraktionsuntereinheit 30i eingerichtet, aus den selektierten Bewegungssignalen 8 mindestens ein Bewegungsmerkmal 9 und aus den selektierten Herzsignalen 5 mindestens ein Herzmerkmal 6 und aus den selektierten Atmungssignalen 2 mindestens ein Atmungsmerkmal 3 zu ermitteln. Die erste Extraktionsuntereinheit 30i kann demnach aus drei Signalen 2,5,8 unterschiedlicher Kategorien (körperliche Bewegung; Herztätigkeit; Atmungstätigkeit) jeweils mindestens ein Merkmal 3,6,9 extrahieren. Die erste Extraktionsuntereinheit 30i kann auch jeweils mehr als ein Merkmal 3,6,9 extrahieren.

In dem konkreten Ausführungsbeispiel nach Fig. 4 ist die zweite Extraktionsuntereinheit 30ii eingerichtet, aus den selektierten Bewegungssignalen 8 mindestens ein Bewegungsmerkmal 9 und aus den selektierten Herzsignalen 5 mindestens ein Herzmerkmal 6 zu ermitteln. Die zweite Extraktionsuntereinheit 30ii kann demnach aus zwei Signalen 5,8 unterschiedlicher Kategorien (körperliche Bewegung; Herztätigkeit) jeweils mindestens ein Merkmal 6,9 extrahieren. Die zweite Extraktionsuntereinheit 30ii kann auch jeweils mehr als ein Merkmal 3,6 extrahieren.

In dem konkreten Ausführungsbeispiel nach Fig. 4 ist die dritte Extraktionsuntereinheit 30iii eingerichtet, aus den selektierten Bewegungssignalen 8 mindestens ein Bewegungsmerkmal 9 zu ermitteln. Die dritte Extraktionsuntereinheit 30iii kann demnach aus einem Signalen 8 (körperliche Bewegung) mindestens ein Merkmal 9 extrahieren. Die dritte Extraktionsuntereinheit 30iii kann aus den Signalen 8 auch mehr als ein Merkmal 9 extrahieren.

Die Ansteuerung der Untereinheiten 30i, 30ii, 30iii kann durch die Überwachungseinheit 20 erfolgen. Die Steuerung kann auch durch die Extraktionseinheit 30 selbst erfolgen. In einer beispielhaften Ausführungsform ist vorgesehen, dass die übergeordnete Steuereinheit 101 registriert, um welche Kombination an Signalen 2, 5, 8 es sich handelt und daraufhin die richtige Untereinheit 30i, 30ii oder 30iii ansteuert. In jedem Falle erhält die jeweilige Untereinheit 30i, 30 ii oder 30iii die entsprechenden Signale 2 und/oder 5 und/oder 8 zur Weiterverarbeitung.

Die unterschiedlichen Extraktionseinheiten 30i, 30 ii,30iii können während eines Zeitraum 112 einmalig und/oder mehrmals angesteuert werden, nämlich in Abhängigkeit von dem - von der Überwachungseinheit 20 ermittelten - Vorhandensein und der Qualität der Signale 2,5,8. Bevorzugt erfolgt die Ansteuerung und somit die Auswahl der entsprechenden Untereinheit 30i, 30ii, 30iii für jede einzelne Zeitperiode 113 von neuem.

Das System 100 kann in Zusammenhang mit einem Beatmungsgerät 90 betrieben werden (nicht gezeigt). Das Beatmungsgerät 90 kann eingerichtet sein, einen Patienten oder sonstigen Anwender bei der natürlichen Atmung unterstützen und/oder die Beatmung eines Anwenders oder Patienten übernehmen und/oder einer Atemtherapie dienen und/oder anderweitig auf die Atmung eines Anwenders oder Patienten einwirken. Das Beatmungsgerät 90 kann für klinische oder Heimanwendungen eingerichtet sein. Das Beatmungsgerät 90 kann insbesondere dazu eingerichtet und ausgebildet sein eine CPAP- und/oder APAP-Therapie durchzuführen.

Das Beatmungsgerät 90 weist eine Schnittstelle zum Koppeln eines Schlauchsystems 94 auf, über das dem Patienten oder Anwender eine Atemluftströmung zur Beatmung oder Atemunterstützung zugeführt werden kann. Zu diesem Zwecke kann an das Schlauchsystem 94 ein Patienteninterface 92 angeschlossen werden. Als Patienteninterface 92 ist jegliches Peripheriegerät zu verstehen, welches zur Interaktion mit einem Lebewesen ausgebildet ist. Insbesondere ist das Patienteninterface 92 zu Therapie- und/oder Diagnosezwecken in Verbindung mit dem Beatmungsgerät 90 ausgebildet. Das Patienteninterface 92 kann als Atemmaske ausgebildet sein, zum Beispiel als Nasenbrille bzw. Sauerstoffbrille, Nasenmaske, Nasenpolstermaske, Full-Face- oder Totalface-Maske sowie Trachealtuben bzw. -kanülen. Im oder am oder in Verbindung mit dem Patienteninterface 92 kann der Sensor 1 zur Messung des Atmungssignals 2 angeordnet sein.

Nachfolgend werden Beispiele beschrieben für
- aus den Atmungssignalen 2 extrahierte Atmungsmerkmale 3
- aus den Herzsignalen 5 extrahierte Herzmerkmale 6
- aus den Bewegungssignalen 8 extrahierte Bewegungsmerkmale 9.

Die Erfindung ist nicht auf die beschriebenen Merkmale 3,6,9 beschränkt.

Aus den Atmungssignalen 2 (siehe Fig. 2) abgeleitet werden können Atmungsmerkmale 3 ausgewählt aus der Gruppe: Atemfrequenz, Peakflow, Variabilität, Atemgasvolumen pro Atemzug, Atemgasfluss, Kontur des Atemgasflusses, inspiratorisches oder exspiratorisches Tidalvolumen, Atemzugsdauer, Inspirationsdauer, Exspirationsdauer, Leckage, Verhältnis von Inspirations- zu Exspirationsdauer, Atemminutenvolumen.

Aus den Bewegungssignalen 8 (siehe Fig. 2) abgeleitet werden können Bewegungsmerkmal 9 ausgewählt aus der Gruppe: Bewegungszählung (Activity count), Nulldurchgangszählung (Zero-Crossing count), Median, Standardabweichung, Beschleunigungs-Minima, Beschleunigungs-Maxima.

Aus den PPG-Signalen 5 (siehe Fig. 2) abgeleitet werden können Herzmerkmale 6 ausgewählt aus der Gruppe: Herzfrequenz, Herzschlag, Herzschlagintervall, Herzfrequenzvariabilität, Pulsfrequenz, Pulswelle, Pulswellenamplitude, Spektrale Leistungsdichte, Sauerstoffsättigung (SpO₂ oder SaO₂), Plethysmogramm, Anstiegswinkel der Pulswellen, Abfallswinkel der Pulswellen, Verhältnisse aus Anstiegs- zu Abfallwinkel der Pulswellen, Dauern von Pulswellenanstiegen, Dauern von Pulswellenabfällen, Verhältnisse von Anstiegs- zu Abfallsdauern, Pulswellenmaxima, Pulswellenminima, PTT (Pulstransit-Zeit), CWF (continous wave fluctuation), Pulsamplitude.

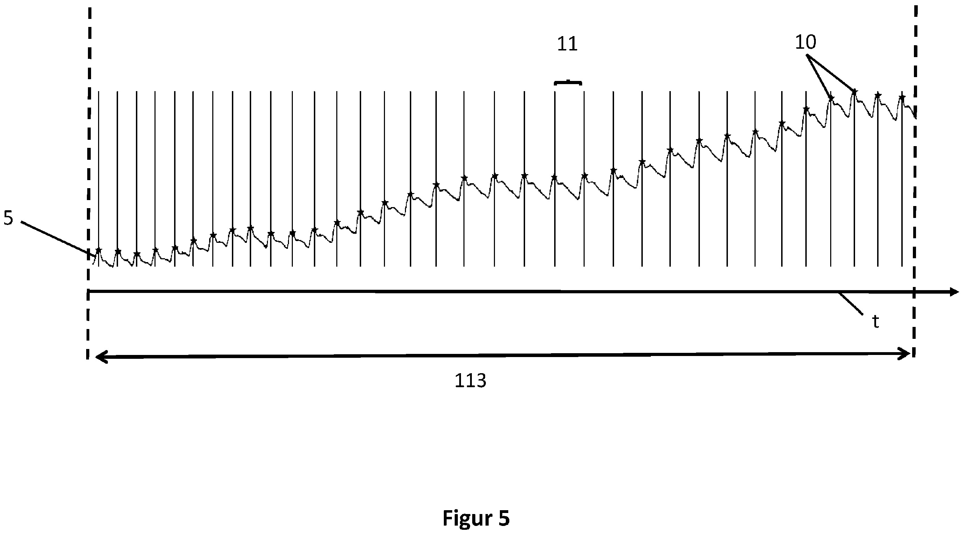

**Fig. 5** zeigt ein beispielhaftes ermitteltes (Photo-)Plethysmogramm über die Zeit t mit einer beispielhaften Darstellung extrahierter Herzmerkmale 6.

Die PPG-Signale 5 können in einem Plethysmogramm dargestellt werden. In Fig. 5 ist beispielhaft der Verlauf des PPG-Signals 5 über die Zeit t in einer beispielhaften Zeitperiode 113 dargestellt. Beispielsweise anhand des Plethysmogramms können ein oder mehrere Herzmerkmale 6 extrahiert werden. In der Extraktionseinheit 30 kann zu diesem Zweck wenigstens ein Algorithmus hinterlegt sein, mittels dem aus einem oder mehreren Herzsignalen 5 mindestens ein Herzmerkmal 6 berechnet werden kann. Der Algorithmus kann eingerichtet sein, eine Merkmalsdetektion durchzuführen. Die PPG-Signale 5 haben ein periodisches Muster. Der Algorithmus kann eingerichtet sein, aus dem periodischen Muster mindestens ein Herzmerkmal 6 zu berechnen bzw. abzuleiten. Es ist auch möglich, anhand des Plethysmogramms mehrere Herzmerkmale 6 abzuleiten.

Beispielsweise erfolgt eine Merkmalsdetektion aus den PPG-Signalen 5 anhand einer Detektion von Peaks (Höhepunkten) 10 im PPG-Verlauf (siehe Fig. 5). Hierfür kann ein Algorithmus für eine Detektion der Peaks 10 hinterlegt sein. Die Peaks 10 stellen den Herzschlag des Patienten dar. Da diese Peaks 10 dem Herzschlag 10 entsprechen, können aus den Peaks 10 unter anderem die Pulswelle und/oder die Herzfrequenz 11 und/oder die Herzfrequenzvariabilität und dergleichen abgeleitet werden.

Beispielsweise kann die Herzfrequenz 11 aus den detektierten Peaks 10 ermittelt werden. Die Herzfrequenz 11 ist der Kehrwert des Abstandes zwischen zwei aufeinanderfolgenden Peaks 10. Wenn beispielsweise der Abstand zwischen zwei aufeinanderfolgenden Peaks 10 1,5 Sekunden beträgt, liegt die Herzrate bei 1/1,5 Hz = 60/1,5 Schläge pro Minute.

Alternativ oder zusätzlich kann eine Variabilität der Herzfrequenz (heart rate variability, HRV) erfasst werden, also die Variation der Zeit t zwischen zwei aufeinanderfolgenden Herzschlägen 10.

Der Algorithmus kann somit eingerichtet sein, Herzmerkmale 6 wie beispielsweise den Herzschlag 10 und/oder die Herzfrequenz 11 und/oder das Herzschlagintervall (beat-to-beat-interval, BBI) und/oder die Variabilität der Herzfrequenz und/oder die Pulsamplitude und/oder andere Herzmerkmale 6 zu ermitteln.

Diese Herzmerkmale 6 können in der Extraktionseinheit 30 und/oder in der Klassifikationseinheit 40 statistisch ausgewertet werden. Beispielsweise können die Maxima, Minima, Mittelwerte, Mediane, Standardabweichungen, Varianzen, Spannweiten, Verteilungen, Summen oder Differenzen des einen oder der mehreren Herzmerkmale 6 ermittelt werden. Die extrahierten Herzmerkmale 6 und/oder deren statistischen Auswertungen können eine Grundlage für die Ermittlung mindestens einer Schlafphase 110 und/oder einer Wachphase 111 darstellen.

Anhand der extrahierten Herzmerkmale 6 und/oder deren statistischen Auswertung kann auch ein Rückschluss auf die Qualität der PPG-Aufzeichnung gezogen werden. Wenn Herzmerkmale 6 wie Herzfrequenz 11 oder Pulsamplitude außerhalb des physiologischen Bereiches liegen, wird dem PPG eine schlechte Qualität zugeordnet. Ein Rückschluss auf die Qualität des PPG-Signals 5 kann auch von der absoluten Höhe des PPG-Signals 5 abgeleitet werden. Eine schnelle und/oder abrupte Änderung der absoluten Höhe des PPG-Signals 5 weist auf eine schlechte Qualität des PPGs hin. Die Qualität der Herzsignale 5 kann somit nicht nur in der Überwachungseinheit 20 erfolgen, sondern auch nachgeschaltet in der Extraktionseinheit 30 und/oder der Klassifizierungseinheit 40.

Um Störungen und/oder singuläre Ereignisse zu eliminieren, kann die Extraktionseinheit 30 bzw. ein in der Extraktionseinheit 30 hinterlegter Algorithmus eine Artefakterkennung und/oder eine Artefaktbeseitigung durchführen.

Eine Steigerung der Messempfindlichkeit kann zudem dadurch erreicht werden, dass die Signalauswertung innerhalb mindestens eines vorgebaren Frequenzbandes durchgeführt wird. Beispielsweise können die PPG-Signale 5 Bandpass gefiltert werden, so dass nur Signale 5 eines vordefinierten Frequenzbandes ausgewertet werden. Die Merkmale im Frequenzbereich, einschließlich der spektralen Leistungsdichte, die schnelle oder kleine Änderungen der HRV-Werte anzeigt, werden ebenfalls berücksichtigt.

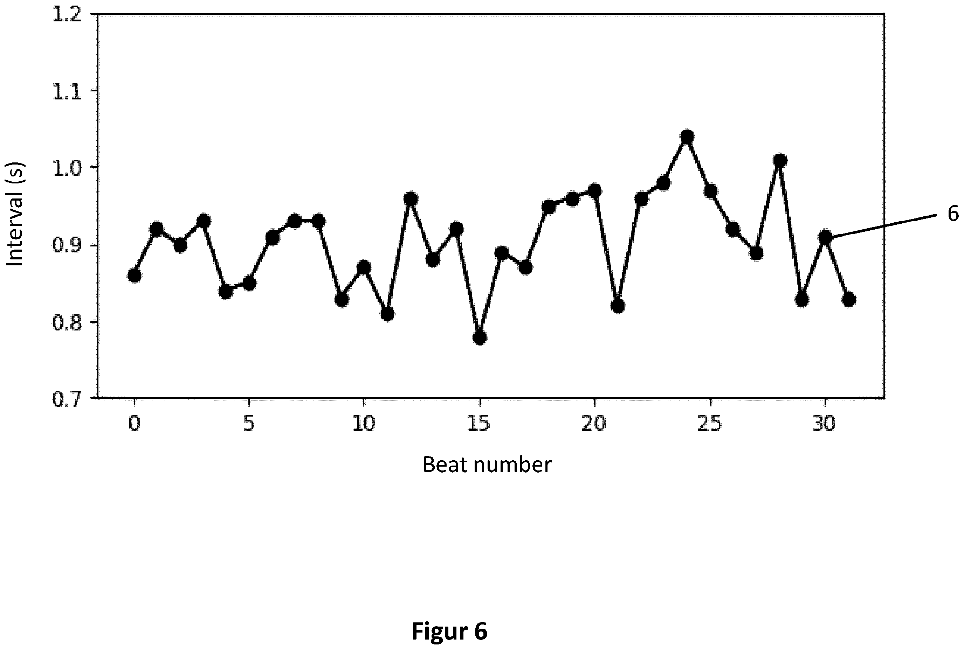

**Fig. 6** zeigt eine beispielhafte Darstellung eines extrahierten Herzmerkmals 6 in einem Koordinatensystem, wobei auf der X-Achse die Herzschläge 10 aufgetragen sind und auf der Y-Achse das Herzschlagintervall in Sekunden. Das Herzschlagintervall und/oder die Variabilität des Herzschlagintervalls kann als Herzmerkmal 6 ermittelt werden und beispielsweise Rückschlüsse auf Schlafphasen 110 und/oder Wachphasen 111 bieten.

Anhand eines solchen in Fig. 6 dargestellten Kardiointervallograms/Rythmogramms können beispielsweise Rückschlüsse über den vegetativen Regulationszustand gezogen werden. Eine dominante Sinus-Atmungsarrythmie weist beispielsweise darauf hin, dass sich der Patient in relativer Ruhe befindet, was zum Beispiel einer Schlafphase 110 entsprechen kann.

Wie bereits weiter oben hierin ausgeführt, kann das System 100 dazu eingerichtet und ausgebildet sein, anhand der Bewegungssignale 8 und/oder der Herzsignale 5 und/oder der Atmungssignale 2 mindestens einer Zeitperiode 113 eine Schlafphase 110 und/oder eine Wachphase 111 zuzuordnen.

In einem bevorzugten Ausführungsbeispiel kann das System 100 auch dazu eingerichtet und ausgebildet sein, anhand der Bewegungsmerkmale 9 und/oder der Herzmerkmale 6 und/oder der Atmungsmerkmale 3 mindestens einer, bevorzugt jeder analysierten Zeitperiode 113 eine Schlafphase 110 und/oder eine Wachphase 111 zuzuordnen.

Bevorzugt ordnet das System 100 jeder Zeitperiode 113 des Zeitraumes 112 eine Schlafphase 110 und/oder eine Wachphase 111 zu. Durch die Auswahl aus den Kategorien körperliche Bewegung, Herztätigkeit und Atmungstätigkeit stehen erfindungsgemäß stets ausreichende Signale 2,5,8 bzw. Merkmale 3,6,9 zur Verfügung, um bevorzugt jeder Zeitperiode 113 eine Schlafphase 110 und/oder eine Wachphase 111 zuzuordnen.

In einer einfachen Ausführungsform können die selektierten Bewegungssignale 8 und/oder Herzsignale 5 und/oder Atmungssignale 2 direkt an die Klassifizierungseinheit 40 weitergeleitet werden (siehe Fig. 3). In einer bevorzugten Ausführungsform können die Signale 8,5,2 zunächst von der Extraktionseinheit 30 derart verarbeitet werden, dass die Klassifizierungseinheit 40 extrahierte Merkmale 3,6,9 und/oder deren statistische Auswertungen empfängt (siehe Fig. 4).

Die Klassifizierungseinheit 40 kann mehrere Klassifizierungsuntereinheiten umfassen. Beispielsweise umfasst die Klassifizierungseinheit 40 drei Klassifizierungsuntereinheiten 40i, 40ii, 40iii. Die Steuereinheit 101 kann eingerichtet sein, basierend auf den Ergebnissen der Überwachungseinheit 20 in Abhängigkeit von dem Vorhandensein und/oder der Qualität der Signale 2, 5, 9 jeweils eine der drei Klassifizierungsuntereinheiten 40i, 40ii, 40iii anzusteuern.

Die erste Klassifizierungsuntereinheit 40i kann angesteuert werden, wenn alle Signale 2,5,9 in ausreichender Qualität vorhanden sind. zweite Die Klassifizierungsuntereinheit 40ii kann angesteuert werden, wenn nur die Signale 5 und 9 in ausreichender Qualität vorhanden sind. Die dritte Klassifizierungsuntereinheit 40iii kann angesteuert werden, wenn nur die Signale 9 in ausreichender Qualität vorhanden sind.

Die erste Klassifizierungsuntereinheit 40i kann sodann eingerichtet sein, Merkmale aller drei verschiedenen Kategorien zu empfangen. Die erste Klassifizierungsuntereinheit 40i kann also eingerichtet sein, Bewegungsmerkmale 9 und Herzmerkmale 6 und Atmungsmerkmale 3 zu empfangen und Schlafphasen 110 und/oder Wachphasen 111 aus den Bewegungsmerkmalen 9 und den Herzmerkmalen 6 und den Atmungsmerkmalen 3 zu ermitteln.

Die zweite Klassifizierungsuntereinheit 40ii kann eingerichtet sein, Merkmale von zwei verschiedenen Kategorien zu empfangen. Die zweite Klassifizierungsuntereinheit 40ii kann also eingerichtet sein, Bewegungsmerkmale 9 und Herzmerkmale 6 zu empfangen und Schlafphasen 110 und/oder Wachphasen 111 aus den Bewegungsmerkmalen 9 und den Herzmerkmalen 6 zu ermitteln.

Die dritte Klassifizierungsuntereinheit 40iii kann eingerichtet sein, Merkmale einer einzigen Kategorie zu empfangen. Das Merkmal das alleine zur Verfügung stehen kann ist in der Regel aus der Kategorie körperliche Bewegung. Die dritte Klassifizierungsuntereinheit 40iii kann somit insbesondere eingerichtet sein, Bewegungsmerkmale 9 zu empfangen und Schlafphasen 110 und/oder Wachphasen 111 aus den Bewegungsmerkmalen 9 zu ermitteln.

In alternativen, hier nicht gezeigten Ausführungsbeispielen kann das System 100 auch weitere Klassifizierungsuntereinheiten umfassen, die die übrigen Kombinationen an Merkmalen verwerten können nämlich beispielweise eine Kombination aus Bewegungsmerkmalen 9 und Atmungsmerkmalen 3 oder eine Kombination aus Herzmerkmalen 6 und Atmungsmerkmalen 3 oder alleinige die Atmungsmerkmale 3 oder alleinig die Herzmerkmale 6, um daraus Schlafphasen 110 und/oder Wachphasen 111 zu ermitteln.

Die Klassifizierungseinheit 40 bzw. ihre Untereinheiten sind demnach eingerichtet, Signale 2, 5, 8 von der Überwachungseinheit 20 und/oder Merkmale 3, 6, 9 von den Extraktionseinheiten 30 zu empfangen. In der Klassifizierungseinheit 40 kann sodann die Auswertung der Signale 2,5,8 und/oder der Merkmale 3,6,9 erfolgen. Zu diesem Zwecke ist in der Klassifizierungseinheit mindestens ein Algorithmus zur Erkennung von Schlafphasen 110 bzw. Wachphasen 111 hinterlegt, der die Signale 2,5,8 und/oder der Merkmale 3,6,9 analysiert.

Die Auswertung der extrahierten Signale 2,5,8 und/oder Merkmale 3,6,9 kann über eine Kalkulation der Statistik anhand mindestens einer statistischen Kennzahl 46 erfolgen. Beispielsweise können Minima, Maxima, Mittelwerte, Mediane, Standardabweichungen, Varianzen, Spannweiten, Verteilungen, Summen oder Differenzen der Signale 2,5,8 und/oder Merkmale 3,6,9 ermittelt werden. Bevorzugt werden die Merkmale 3,6,9 statistisch ausgewertet.

In der Klassifizierungseinheit 40 kann mindestens ein Grenzwert 42 hinterlegt sein, anhand dessen die Zuordnung der Schlafphasen 110 und/oder Wachphasen 111 zu den Zeitperioden 113 ermittelt wird. Einer Zeitperiode 113 kann eine Schlafphase 110 zugeordnet werden, wenn die analysierten Werte (Signale 2,5,8 und/oder Merkmale 3,6,9) über dem Grenzwert 42 liegen. Im Umkehrschluss kann einer Zeitperiode 113 eine Wachphase 111 zugeordnet werden, wenn die analysierten Werte (Signale 2,5,8 und/oder Merkmale 3,6,9) unter dem Grenzwerten 42 liegen und vice versa. Bevorzugt wird jedem analysierten Wert, also jedem analysiertem Signal 2,5,9 bzw. Merkmal 3,6,9 jeweils ein eigener Grenzwert 42 zugeordnet. In der Klassifizierungseinheit 40 können somit mehrere Grenzwerte 42 hinterlegt und abrufbar sein.

Die Analyse der unterschiedlichen Werte, also der Signale 2,5,8 und/oder Merkmale 3,6,9 kann unterschiedlich gewichtet werden. Zu diesem Zweck kann in der Klassifizierungseinheit 40 mindestens ein Gewichtwert 44 hinterlegt und abrufbar sein. In einem bevorzugten Ausführungsbeispiel kann für jedes einzelne Signal 2,5,8 und/oder Merkmal 3,6,9 ein jeweiliger Gewichtwert 44 hinterlegt sein.

Das Ziel ist es, jeder Zeitperiode 113 des Zeitraumes 112 ein Label "Schlafphase 110" oder "Wachphase 111" zuzuordnen. Das erfindungsgemäße System 100 ist eingerichtet, einen Algorithmus zur Erkennung von Schlafphasen 110 und Wachphasen 111 basierend auf der Analyse der Atmungsmerkmale 3 und/oder Herzmerkmale 6 und/oder Bewegungsmerkmale 9 sowie weiteren Daten/Werten beispielsweise in Form von Gewichtwerten 44 und Grenzwerten 42 auszuführen.

Die Erkennung einer "Schlafphase 110" oder einer "Wachphase 111" wird zum Beispiel durch einen Machine-Learning Algorithmus ausgeführt. Dazu werden initial empirisch ermittelte Daten von Lebewesen hinsichtlich der Schlafphasen 110 oder Wachphasen 111 manuell ausgewertet und dem Machine-Learning Algorithmus zur Verfügung gestellt.

In der Machine-Learning Phase werden also im Vorfelde bestimmten empirisch ermittelten Daten (der Auswertung von Merkmalen oder Merkmalskombinationen) ein jeweiliges Label "Schlafphase 110" oder "Wachphase 111" zugeordnet. Der Machine-Learning Algorithmus kann dann auf neu ermittelte, unbekannte Daten angewendet werden.

Beispielsweise wird der Machine-Learning Algorithmus nach Anlernen durch die manuell ausgewerteten Daten im System 100, beispielsweise in der Klassifizierungseinheit 40, gespeichert und ausgeführt.

Der Algorithmus kann die unterschiedlichen Kombinationen verschiedener Atmungsmerkmale 3, Herzmerkmale 6 und Bewegungsmerkmale 9 und ihrer jeweiligen Gewichtwerte 44 mit einem Grenzwert 42 vergleichen. Es kann somit die Summe aus (*Merkmal A^{∗}Gewichtwert A* + *Merkmal B ^{∗} Gewichtwert B +* ...) mit einem jeweiligen Grenzwert 42 verglichen werden. Die Buchstaben A, B ... stehen hierbei jeweils für ein spezifisches ermitteltes Atmungsmerkmal 3 oder Herzmerkmal 6 oder Bewegungsmerkmal 9.

Die Gewichtwerte 44 können im Vorfeld in einer Maschinenlernen-Phase von dem Machine-Learning Algorithmus ermittelt und hinterlegt werden. Die Maschinenlernen-Werkzeuge werden als "Black-Box" genutzt, in die Daten (Merkmale und Label) sowie empirisch ermittelte Parameter eingegeben werden können. Dadurch lernt bzw. ermittelt die "Black-Box" die Gewichtwerte 44 für jedes einzelne Merkmal 3,6,9. Die ermittelten Gewichtwerte 44 und Grenzwerte 42 können sodann im Algorithmus verwendet werden.

Die Berechnung, ob in einer Zeitperiode 113 eine Schlafphase 110 oder eine Wachphase 111 besteht, kann als lineares Modell und/oder als Entscheidungsbaum erfolgen. Bei einem linearen Modell werden im Zeitraum 112 (also während der Laufzeit) für jede Zeitperiode 113 das mindestens eine Merkmal berechnet und mit den zuvor eingespeisten Gewichtwerten 44 verrechnet. Anhand der oben genannten linearen Formel "Merkmal A^{∗}Gewichtwert A + Merkmal B ^{∗} Gewichtwert B + ..." wird das Ergebnis mit einem zuvor festgelegten Grenzwert 42 verglichen. Derart kann eine Entscheidung getroffen werden kann, ob eine Schlafphase 110 oder eine Wachphase 111 besteht.

Bei einem Entscheidungsbaum kann ein im Vorfelde festgelegter Binärbaum mit mindestens einem Knotenpunkt verwendet werden. An den Knotenpunkten sind die jeweiligen Grenzwerte 42 hinterlegt, anhand derer die Entscheidung getroffen werden kann, ob eine Schlafphase 110 oder eine Wachphase 111 besteht. Wenn das Merkmal X kleiner als der zugehörige Grenzwert X ist, wird ein erster Weg eingeschlagen und wenn das Merkmal X größer als der zugehörige Grenzwert X ist, wird ein zweiter Weg eingeschlagen. Jeder Weg hat am Ende das Label "Schlafphase 110" oder "Wachphase 111". Während der Laufzeit können die Merkmale für jede neue Zeitperiode 113 berechnet werden. Dann traversieren wir den Baum und landen bei einem Label "Schlafphase 110" oder einem Label "Wachphase 111".

Die Einheiten 20,30,40 sind somit als Recheneinheiten 20, 30, 40 eingerichtet und ausgebildet, in denen Algorithmen mit hinterlegten Schwellenwerten 22 und/oder Grenzwerten 42 und/oder Gewichtwerten 44 hinterlegt sind. Diese Schwellenwerte 22 und/oder Grenzwerte 42 und/oder Gewichtwerte 44 werden auf Grundlage zurückliegender Zeitperioden 113 bzw. Zeiträume 112 in einer Designzeit des Systems 100 ermittelt.

Es ist denkbar, dass der Machine-Learning Algorithmus des Systems 100 Lernfortschritte erzielt und diese über die Schnittstelle 65 an einen Server bzw. eine Cloud sendet. Somit können die Lernfortschritts-Daten gesammelt und genutzt werden, den Algorithmus zu verbessern bzw. zu optimieren oder zu aktualisieren. Dies geschieht außerhalb der Laufzeit, also nicht während einer Zeitperiode 112. So kann es auch eingerichtet sein, dass das System 100 von Zeit zu Zeit mit Daten vom Server aktualisiert wird und/oder weitere Lerndaten zur Verfügung gestellt werden. Der Machine-Learning Algorithmus kann stets oder periodisch weiterlernen, beispielsweise durch weitere manuell ausgewertete Daten.

Ferner zeigen Fig. 3 und Fig. 4 beispielhafte Abläufe des erfindungsgemäßen Verfahrens zur Ermittlung von mindestens einer Schlafphase 110 und/oder einer Wachphase 111 für mindestens einen Zeitraum 112. In einem ersten Schritt wird erfindungsgemäß mindestens ein Signal mindestens eines Bewegungsparameters 8 und/oder mindestens ein Signal mindestens eines Herzparameters 5 und/oder mindestens eines Signals mindestens eines Atmungsparameters 2 erfasst. In einem weiteren Schritt wird das Vorhandensein der erfassten Bewegungssignale 8 und Herzsignale 5 und Atmungssignale 2und/oder eine Bewertung der Qualität der erfassten Bewegungssignale 8 und Herzsignale 5 und Atmungssignale 2 ermittelt. In einem weiteren Schritt wird mindestens eine Schlafphase 110 und/oder eine Wachphase 111 aus den Bewegungssignalen 8 und/oder den Herzsignalen 5 und/oder den Atmungssignalen 2 ermittelt.

Zu diesem Zwecke kann aus den Bewegungssignalen 8 mindestens ein Bewegungsmerkmal 9 und/oder aus den Herzsignalen 5 mindestens ein Herzmerkmal 6 und/oder aus den Atmungssignalen 2 mindestens ein Atmungsmerkmal 3 ermittelt werden. Sodann kann aus den Bewegungsmerkmalen 9 und/oder den Herzmerkmalen 6 und/oder den Atmungsmerkmalen 3 mindestens eine Schlafphase 110 und/oder eine Wachphase 111 ermittelt werden.

Es ist auch daran gedacht, dass die Steuereinheit 101 eingerichtet und ausgebildet ist, die folgenden Verfahrensschritte zur Ermittlung von mindestens einer Schlafphase 110 und/oder einer Wachphase 111 für mindestens einen Zeitraum 112 auszuführen: Erfassen mindestens eines Signals mindestens eines Bewegungsparameters 8 und/oder Erfassen mindestens eines Signals mindestens eines Herzparameters 5 und/oder Erfassen mindestens eines Signals mindestens eines Atmungsparameters 2, sowie Ermitteln des Vorhandenseins und/oder Bewerten der Qualität der erfassten Bewegungssignale 8 und Herzsignale 5 und Atmungssignale 2, sowie Ermitteln mindestens einer Schlafphase 110 und/oder einer Wachphase 111 aus den Bewegungssignalen 8 und/oder den Herzsignalen 5 und/oder den Atmungssignalen 2 auf Basis der Ermittlungen und Bewertungen der Überwachungseinheit 20.

Obwohl die vorliegende Erfindung anhand der Ausführungsbeispiele detailliert beschrieben wurde, ist es für den Fachmann selbstverständlich, dass die Erfindung nicht auf diese Ausführungsbeispiele beschränkt ist. Vielmehr sind Abwandlungen in einer Weise möglich, dass einzelne Merkmale weggelassen werden oder andersartige Kombinationen der beschriebenen Einzelmerkmale verwirklicht werden können, sofern der Schutzbereich der beiliegenden Ansprüche nicht verlassen wird. Die vorliegende Offenbarung schließt sämtliche Kombinationen der vorgestellten Einzelmerkmale mit ein.

### Bezugszeichenliste

- 1: Sensor (z.B. Flow-Sensor)
- 2: Atmungssignal
- 3: Atmungsmerkmal
- 4: Sensor (z.B. PPG-Sensor)
- 5: Herzsignal
- 6: Herzmerkmal
- 7: Sensor (z.B. Aktigraphie -Sensor)
- 8: Bewegungssignal
- 8X: Bewegungssignal X-Achse
- 8Y: Bewegungssignal Y-Achse
- 8Z: Bewegungssignal Z-Achse
- 9: Bewegungsmerkmal
- 10: Herzschlag (Peak)
- 11: Herzfrequenz (Beat-to-Beat-Interval)
- 20: Überwachungseinheit
- 22: Schwellenwert
- 30: Extraktionseinheit
- 30i: Erste Extraktionsuntereinheit
- 30ii: Zweite Extraktionsuntereinheit
- 30iii: Dritte Extraktionsuntereinheit
- 40: Klassifizierungseinheit
- 40i: Erste Klassifizierungsuntereinheit
- 40ii: Zweite Klassifizierungsuntereinheit
- 40iii: Dritte Klassifizierungsuntereinheit
- 42: Grenzwert
- 44: Gewichtwert
- 46: Statistische Kennzahl
- 60: Speichereinheit
- 62: Energieversorgung
- 64: Datenübertragungseinrichtung
- 65: Schnittstelle
- 66: Anzeigeeinrichtung
- 80: Vorrichtung
- 82: Vorrichtungsgurt
- 84: Kabelverbindung
- 85: Anschluss
- 86: Schlauchverbindung
- 87: Anschluss
- 90: Beatmungsgerät
- 92: Patienteninterface
- 94: Schlauchsystem
- 100: System
- 101: Steuereinheit
- 110: Schlafphase
- 111: Wachphase
- 112: Zeitraum
- 113: Zeitperiode
- 120: Schlafqualität
- t: Zeit

## Patentansprüche

1. System 100 mit einer Steuereinheit 101 zur Ermittlung von mindestens einer Schlafphase 110 und/oder einer Wachphase 111 für mindestens einen Zeitraum 112 umfassend
- mindestens einen Sensor 7 eingerichtet zur Erfassung mindestens eines Bewegungssignals 8,
- mindestens einen Sensor 4 eingerichtet zur Erfassung mindestens eines Herzsignals 5,
- mindestens einen Sensor 1 eingerichtet zur Erfassung mindestens eines Atmungssignals 2,
- mindestens eine Überwachungseinheit 20, die eingerichtet ist, ein Vorhandensein und/oder eine Qualität der erfassten Bewegungssignale 8 und Herzsignale 5 und Atmungssignale 2 zu ermitteln und/oder zu bewerten,
- eine oder mehrere Klassifizierungseinheiten 40, die eingerichtet sind, auf Basis der Ermittlungen und Bewertungen der Überwachungseinheit 20 aus den Bewegungssignalen 8 und/oder den Herzsignalen 5 und/oder den Atmungssignalen 2 mindestens eine Schlafphase 110 und/oder eine Wachphase 111 zu ermitteln.

2. System 100 nach einem der voranstehenden Ansprüche, **dadurch gekennzeichnet, dass** die Überwachungseinheit 20 eingerichtet ist, Bewegungssignale 8 und/oder Herzsignale 5 und/oder Atmungssignale 2 von den Sensoren 7, 4, 1 zu empfangen, wobei in der Überwachungseinheit 20 mindestens ein Schwellenwert 22 hinterlegt ist und dass die Qualität vorhandener Bewegungssignale 8 und/oder Herzsignale 5 und/oder Atmungssignale 2 anhand des mindestens einen vordefinierten Schwellenwertes 22 bewertet wird.

3. System 100 nach einem der voranstehenden Ansprüche, **dadurch gekennzeichnet, dass** die Überwachungseinheit 20 eingerichtet ist, vorhandene Bewegungssignale 8 und/oder Herzsignale 5 und/oder Atmungssignale 2, die über oder unter dem jeweiligen Schwellenwert 22 liegen, zu selektieren und/oder weiterzuleiten.

4. System 100 nach einem der voranstehenden Ansprüche, **dadurch gekennzeichnet, dass** das System 100 eine oder mehrere Extraktionseinheiten 30 umfasst, die eingerichtet sind, selektierte Signale 2, 5, 8 von der Überwachungseinheit 20 zu empfangen und aus den selektierten Bewegungssignalen 8 mindestens ein Bewegungsmerkmal 9 und/oder aus den selektierten Herzsignalen 5 mindestens ein Herzmerkmal 6 und/oder aus den selektierten Atmungssignalen 2 mindestens ein Atmungsmerkmal 3 zu ermitteln und/oder weiterzuleiten.

5. System 100 nach einem der voranstehenden Ansprüche, **dadurch gekennzeichnet, dass** die ermittelten Bewegungsmerkmale 9 ausgewählt sind aus der Gruppe Bewegungszählung (Activity count), Nulldurchgangszählung (Zero-Crossing count), Median, Standardabweichung, Beschleunigungs-Minima, Beschleunigungs-Maxima.

6. System 100 nach einem der voranstehenden Ansprüche, **dadurch gekennzeichnet, dass** die ermittelten Herzmerkmale 6 ausgewählt sind aus der Gruppe Herzfrequenz, Herzschlag, Herzschlagintervall, Herzfrequenzvariabilität, Pulsfrequenz, Pulswelle, Pulswellenamplitude, Spektrale Leistungsdichte, Sauerstoffsättigung (SpO₂ oder SaO₂), Plethysmogramm, Anstiegswinkel der Pulswellen, Abfallswinkel der Pulswellen, Verhältnisse aus Anstiegs- zu Abfallwinkel der Pulswellen, Dauern von Pulswellenanstiegen, Dauern von Pulswellenabfällen, Verhältnisse von Anstiegs- zu Abfallsdauern, Pulswellenmaxima, Pulswellenminima, PTT (Pulstransit-Zeit), CWF (continous wave fluctuation), Pulsamplitude.

7. System 100 nach einem der voranstehenden Ansprüche, **dadurch gekennzeichnet, dass** die ermittelten Atmungsmerkmale 3 ausgewählt sind aus der Gruppe Atemfrequenz, Peakflow, Variabilität, Atemgasvolumen pro Atemzug, Atemgasfluss, Kontur des Atemgasflusses, inspiratorisches oder exspiratorisches Tidalvolumen, Atemzugsdauer, Inspirationsdauer, Exspirationsdauer, Leckage, Verhältnis von Inspirations- zu Exspirationsdauer, Atemminutenvolumen.

8. System 100 nach einem der voranstehenden Ansprüche, **dadurch gekennzeichnet, dass** die Klassifizierungseinheiten 40 eingerichtet sind, Signale 2, 5, 8 von der Überwachungseinheit 20 und/oder Merkmale 3, 6, 9 von den Extraktionseinheiten 30 zu empfangen, wobei die Klassifizierungseinheiten 40 eingerichtet sind, die Signale 2, 5, 8 und/oder die Merkmale 3, 6, 9 anhand mindestens einer statistischen Kennzahl 46 statistisch auszuwerten, wobei die statistische Kennzahl 46 ausgewählt ist aus der Gruppe Minimum, Maximum, Mittelwert, Median, Standardabweichung, Varianz, Spannweite, Verteilung, Summe, Differenz.

9. System 100 nach einem der voranstehenden Ansprüche, **dadurch gekennzeichnet, dass** die Klassifizierungseinheiten 40 eingerichtet sind, aus den Bewegungssignalen 8 und/oder den Herzsignalen 5 und/oder den Atmungssignalen 2 und/oder aus deren statistischen Auswertungen mindestens eine Schlafphase 110 und/oder eine Wachphase 111 zu ermitteln.

10. System 100 nach einem der voranstehenden Ansprüche, **dadurch gekennzeichnet, dass** die Klassifizierungseinheiten 40 eingerichtet sind, aus den Bewegungsmerkmalen 9 und/oder den Herzmerkmalen 6 und/oder den Atmungsmerkmalen 3 und/oder deren statistischen Auswertungen mindestens eine Schlafphase 110 und/oder eine Wachphase 111 zu ermitteln.

11. System 100 nach einem der voranstehenden Ansprüche, **dadurch gekennzeichnet, dass** die Klassifizierungseinheit 40 drei Klassifizierungsuntereinheiten 40i, 40ii, 40iii umfasst, wobei die Steuereinheit 101 eingerichtet ist, basierend auf den Ergebnissen der Überwachungseinheit 20 in Abhängigkeit von dem Vorhandensein und/oder der Qualität der Signale 2, 5, 8 jeweils eine der drei Klassifizierungsuntereinheiten 40i, 40ii, 40iii anzusteuern, wobei eine erste Klassifizierungsuntereinheit 40i angesteuert wird, wenn alle Signale 2,5,8 in ausreichender Qualität vorhanden sind, wobei eine zweite Klassifizierungsuntereinheit 40ii angesteuert wird, wenn nur die Signale 5 und 8 in ausreichender Qualität vorhanden sind, wobei eine dritte Klassifizierungsuntereinheit 40iii angesteuert wird, wenn nur die Signale 8 in ausreichender Qualität vorhanden sind, wobei das System 100 dynamisch ist, wobei die Steuereinheit 101 während eines Zeitraum 112 die Klassifizierungsuntereinheiten 40i, 40ii, 40iii einmalig und/oder mehrmals ansteuert in Abhängigkeit von dem Vorhandensein und der Qualität der Signale 2,5,8.

12. System 100 nach einem der voranstehenden Ansprüche, **dadurch gekennzeichnet, dass** die erste Klassifizierungsuntereinheit 40i eingerichtet ist, Bewegungsmerkmale 9 und Herzmerkmale 6 und Atmungsmerkmale 3 zu empfangen und Schlafphasen 110 und/oder Wachphasen 111 aus den Bewegungsmerkmalen 9 und den Herzmerkmalen 6 und den Atmungsmerkmalen 3 zu ermitteln.

13. System 100 nach einem der voranstehenden Ansprüche, **dadurch gekennzeichnet, dass** die zweite Klassifizierungsuntereinheit 40ii eingerichtet ist, Bewegungsmerkmale 9 und Herzmerkmale 6 zu empfangen und Schlafphasen 110 und/oder Wachphasen 111 aus den Bewegungsmerkmalen 9 und den Herzmerkmalen 6 zu ermitteln.

14. System 100 nach einem der voranstehenden Ansprüche, **dadurch gekennzeichnet, dass** die dritte Klassifizierungsuntereinheit 40iii eingerichtet ist, Bewegungsmerkmale 9 zu empfangen und Schlafphasen 110 und/oder Wachphasen 111 aus den Bewegungsmerkmalen 9 zu ermitteln.

15. System 100 nach einem der voranstehenden Ansprüche, **dadurch gekennzeichnet, dass** das System 100 eingerichtet und ausgebildet ist, anhand der Bewegungssignale 8 und/oder der Herzsignale 5 und/oder der Atmungssignale 2 mindestens einer, bevorzugt jeder Zeitperiode 113 eine Schlafphase 110 und/oder eine Wachphase 111 zuzuordnen.

16. System 100 nach einem der voranstehenden Ansprüche, **dadurch gekennzeichnet, dass** das System 100 eingerichtet und ausgebildet ist, anhand der Bewegungsmerkmale 9 und/oder der Herzmerkmale 6 und/oder der Atmungsmerkmale 3 mindestens einer, bevorzugt jeder analysierten Zeitperiode 113 eine Schlafphase 110 und/oder eine Wachphase 111 zuzuordnen.

17. System 100 nach einem der voranstehenden Ansprüche, **dadurch gekennzeichnet, dass** das System 100 eingerichtet ist, basierend auf der Anzahl der Schlafphasen 110 und/oder der Wachphasen 111 eine Schlafqualität 120 zu ermitteln.

18. Verfahren zur Ermittlung von mindestens einer Schlafphase 110 und/oder einer Wachphase 111 für mindestens einen Zeitraum 112 umfassend die folgenden Verfahrensschritte
- erfassen mindestens eines Signals mindestens eines Bewegungsparameters 8 und/oder
- erfassen mindestens eines Signals mindestens eines Herzparameters 5 und/oder
- erfassen mindestens eines Signals mindestens eines Atmungsparameters 2, sowie
- ermitteln des Vorhandenseins und/oder bewerten der Qualität der erfassten Bewegungssignale 8 und Herzsignale 5 und Atmungssignale 2, sowie
- ermitteln mindestens einer Schlafphase 110 und/oder einer Wachphase 111 aus den Bewegungssignalen 8 und/oder den Herzsignalen 5 und/oder den Atmungssignalen 2 auf Basis der Ermittlungen und Bewertungen der Überwachungseinheit 20.
